# EUROPEAN PATENT SPECIFICATION

(11) **EP 2 203 567 B1**
(45) Date of publication and mention of the grant of the patent: **11.05.2011**
(21) Application number: 08839669.2
(22) Date of filing: 16.10.2008
(51) Int. Cl.: C12Q 1/68

(54) **HIGH RESOLUTION, HIGH THROUGHPUT HLA GENOTYPING BY CLONAL SEQUENCING**
HOCHAUFLÖSENDE HOCHDURCHSATZ-HLA-GENOTYPISIERUNG MITTELS KLONALER SEQUENZIERUNG
GÉNOTYPAGE HLA À HAUTE RÉSOLUTION ET HAUT DÉBIT PAR SÉQUENÇAGE CLONAL

(30) Priority: 16.10.2007 US 980393 P; 03.10.2008 US 245666
(43) Date of publication of application: 07.07.2010
(73) Proprietor: Roche Diagnostics GmbH, 68305 Mannheim (DE); F. Hoffmann-La Roche AG, 4070 Basel (CH)
(72) Inventor: HIGUCHI, Russell, Gene, Alameda, CA 94501 (US); ERLICH, Henry A., Oakland, CA 94602 (US); BENTLEY, Gordon, Alameda, CA 94501 (US)
(86) International application number: PCT/EP2008/008774
(87) International publication number: WO 2009/049889

(56) References cited:
- WO-A-2006/039673
- BUNCE M ET AL: "Phototyping: comprehensive DNA typing for HLA-A, B, C, DRB1, DRB3, DRB4, DRB5 & DQB1 by PCR with 144 primer mixes utilizing sequence-specific primers (PCR-SSP)" TISSUE ANTIGENS, MUNKSGAARD, COPENHAGEN, DK, vol. 46, 1 January 1995 (1995-01-01), pages 355-367, XP008096257 ISSN: 0001-2815
- BINLADEN JONAS ET AL: "The use of coded PCR primers enables high-throughput sequencing of multiple homolog amplification products by 454 parallel sequencing" PLOS ONE, PUBLIC LIBRARY OF SCIENCE, SAN FRANCISCO, CA, US, vol. 2, no. 2, 1 January 2007 (2007-01-01), pages e197.1-e197.9, XP002482385 ISSN: 1932-6203
- LI A X ET AL: "Multiplexed analysis of polymorphisms in the HLA gene complex using bead array chips" TISSUE ANTIGENS, MUNKSGAARD, COPENHAGEN, DK, vol. 63, no. 6, 1 June 2004 (2004-06-01), pages 518-528, XP002453339 ISSN: 0001-2815

## Description

### BACKGROUND OF THE INVENTION

The HLA class I and class II loci are the most polymorphic genes in the human genome, with a complex pattern of patchwork polymorphism localized primarily in exon 2 for the class II genes and exons 2 and 3 for the class I genes. For current HLA typing methods, allele level resolution of HLA alleles, which is clinically important for hemapoetic stem cell transplantation, is technically challenging. Several large-scale studies have demonstrated that precise, allele-level HLA matching between donor and patient significantly improves overall transplant survival by reducing the incidence and severity of both acute and chronic graft versus host disease and improving the rates of successful engraftment. When, for example, 8 of 8 of the most significant HLA loci are matched vs 6 of 8, survival after transplant was enhanced by 60% after 12 months.

It is current practice to maintain bone marrow donor registries in which millions of potential donors are HLA typed at low-medium resolution for the A, B, and, in many cases, the DRB1 loci. Multiple potentially matched unrelated donors are selected, based on this initial typing, and then typed at allele level resolution at these and additional loci to identify the donor best matched to the recipient.

To date, the highest resolution HLA typing is obtained with fluorescent, Sanger-based DNA sequencing using capillary electrophoresis. However, ambiguities in the HLA typing data can persist due to multiple polymorphisms between alleles and the resultant phase ambiguities when both alleles are amplified and sequenced together. Resolving these ambiguities requires time-consuming approaches such as amplifying and then analyzing the two alleles separately.

Next-generation sequencing methods clonally propagate in parallel millions of single DNA molecules which are then also sequenced in parallel. Recently, the read lengths obtainable by one such next generation pyrosequencing sequencing method (454 Life Sciences, Inc.) has increased to > 250 nucleotides. The current invention provides improved HLA genotyping methods that are based on the discovery that clonal sequencing can be used for setting the phase of the linked polymorphisms within an exon and makes possible the unambiguous determination of the sequence of each HLA allele.

### BRIEF SUMMARY OF THE INVENTION

The invention is based, in part, on the discovery that an 8-loci HLA genotyping can be performed on samples obtained from multiple subjections in a single sequencing run. In some embodiments, the invention therefore provides a method of determining the HLA genotypes for the HLA genes HLA-A, HLA-B, HLA-C, DRB1, DQA1, DQB1, DPA1, and DPB1 for more than one individual in parallel, the method comprising:
(a) for each individual, amplifying the exons of the HLA-A, HLA-B, HLA-C, DRB1, DQA1, DQB1, DPA1, and DPB1 genes that comprises polymorphic sites to obtain HLA-A, HLA-B, HLA-C, DRB1, DQA1, DQB1, DPA1, and DPB1 amplicons for each individual, wherein each amplification reaction is performed with a forward primer and a reverse primer to amplify an HLA gene exon, where:
   (i) the forward primer comprises the following sequences, from 5' to 3': an adapter sequence, a molecular identification sequence, and an HLA sequence; and
   (ii) the reverse primer comprises the following sequences, from 5' to 3': an adapter sequence, a molecular identification sequence, and an HLA sequence;
(b) pooling HLA amplicons from more than one individual and performing emulsion PCR;
(c) determining the sequence of the HLA-A, HLA-B, HLA-C, DRB1, DQA1, DQB1, DPA1, and DPB I amplicon for each individual using pyrosequencing in parallel; and
(d) assigning the HLA alleles to each individual by comparing the sequence of the HLA amplicons to the known HLA sequence to determine which HLA alleles are present in the individual. In preferred embodiments according to the invention, the forward or reverse primer for amplifying an HLA amplicon has the sequence of an HLA-hybridizing region of a primer set forth in Table 1. Such a primer may additionally comprise the sequence of an adapter region of a primer of Table 1. In further preferred embodiments, the primer may also comprise an individual identification tag of a primer set forth in Table 1. In particular preferred embodiments, the primer has a sequence of a primer set forth in Table 1.

Furthermore, the invention provides a kit comprising primer pairs for obtaining HLA amplicons to determine the HLA genotypes for the HLA genes HLA-A, HLA-B, HLA-C, DRB1, DQA1, DQB1, DPA1, and DPB1 for more than one individual in parallel, wherein the primer pairs comprise a forward primer and a reverse primer to amplify an HLA gene exon, where: (i) the forward primer comprises the following sequences, from 5' to 3': an adapter sequence, a molecular identification sequence, and an HLA sequence; and (ii) the reverse primer comprises the following sequences, from 5' to 3': an adapter sequence, a molecular identification sequence, and an HLA sequence. In preferred embodiments, the kit comprises one ore more of the forward and reverse primers set forth in Table 1. In other preferred embodiments, the kit comprises primer pairs to amplify exons for genotyping HLA genes HLA-A, HLA-B, HLA-C, DRB1, DQA1, DQB1, DPA1, and DPB1, wherein each of the primer pairs is selected from the primers set forth in Table 1.

The invention additionally provides a kit comprising one or more primer pairs, wherein each primer pair comprises a forward primer for obtaining an HLA amplicon that has the sequence of an HLA-hybridizing region of a primer set forth in Table 1; and a reverse primer for obtaining the HLA amplicon that has the sequence of an HLA-hybridizing region of a primer set forth in Table 1. Such a primer additionally comprises an adapter region having a sequence set forth in Table 1. In further preferred embodiments, the primer has an individual identification tag of a primer set forth in Table 1: In particular preferred embodiments, the forward primer has a sequence of a primer set forth in Table 1 and the reverse primer has a sequence of a primer set forth in Table 1.

Furthermore, the invention provides a kit, wherein the kit comprises fifteen HLA primer pairs, where the primer pairs amplify exon 2, exon 3, and exon 4 of HLA-A; exon 2, exon 3, and exon 4 of HLA-B; exon 2, exon 3, and exon 4 of HLA-C; exon 2 of DRB1, exon 2 of DPB1, exon 2 of DPA1, exon 2 of DQA1; and exon 2 and exon 3 of DQB1. In preferred embodiments, the invention provides a kit that comprises at least six of the primer pairs, or at least, eight, nine, ten, eleven, twelve, thirteen, of fourteen of the primer pairs. Preferably, the primer pairs are selected from the primers set forth in Table 1.

In addition, the invention provides a kit, wherein the kit comprises multiple primer pairs for each primer pair that amplifies exon 2, exon 3, and exon 4 of HLA-A; exon 2, exon 3, and exon 4 of HLA-B; exon 2, exon 3, and exon 4 of HLA-C; exon 2 of DRB1 , exon 2 of DPB1, exon 2 of DPA1, exon 2 of DQA1; and exon 2 and exon 3 of DQB1, wherein the multiple primer pairs that amplify an individual exonic region of interest have the same HLA hybridizing region and the same adapter region, but different identification tags. In preferred embodiments, there are 12 or more multiple primer pairs for each exonic region of interest, where the primer pairs have different multiple identification tags.

### BRIEF DESCRIPTION OF THE DRAWINGS

Figure 1 provides a schematic depicting a forward and reverse fusion primer of the invention.
Figure 2 provides a histogram of the read length.
Figure 3 shows the read depth for the total of forward and reverse reads.

### DETAILED DESCRIPTION OF THE INVENTION

The term "allele", as used herein, refers to a sequence variant of a gene. One or more genetic differences can constitute an allele. For HLA alleles, multiple genetic differences typically constitute an allele. Examples of HLA allele sequences are set out in Mason and Parham (1998) Tissue Antigens 51: 417-66, which list HLA-A, HLA-B, and HLA-C alleles and Marsh et al. (1992) Hum. Immunol. 35:1, which list HLA Class II alleles for DRA, DRB, DQA1, DQB1, DPA1, and DPB1.

The terms "polymorphic" and "polymorphism", as used herein, refer to the condition in which two or more variants of a specific genomic sequence, or the encoded amino acid sequence, can be found in a population. A polymorphic position refers to a site in the nucleic acid where the nucleotide difference that distinguishes the variants occurs. As used herein, a "single nucleotide polymorphism", or SNP, refers to a polymorphic site consisting of a single nucleotide position.

The term "genotype" refers to a description of the alleles of a gene or genes contained in an individual or a sample. As used herein, no distinction is made between the genotype of an individual and the genotype of a sample originating from the individual.

As used herein, "determining the genotype" of an HLA gene refers to determining the HLA polymorphisms present in the individual alleles of a subject. In the current invention, "determining the genotype of an HLA-A gene" refers to identifying the polymorphic residues present in at least exon 2 and exon 3, and typically exon 4, at positions that are allelic determinants of an HLA-A gene allele. In the current invention, "determining the genotype of an HLA-B gene" refers to identifying the polymorphic residues present in at least exon 2 and exon 3, and typically exon 4, at positions that are allelic determinants of an HLA-B gene allele; and "determining the genotype of an HLA-C gene" refers to identifying polymorphic residues present in at least exon 2 and exon 3, and typically exon 4, at positions that are allelic determinants of an HLA-C gene. Similarly, in the current invention, "determining the genotype" of a DRB1, DPB1, DPA1, or DQA1 gene refers to identifying the polymorphic residues present in exon 2 at positions that are allelic determinations of said genes and refers to identifying the polymorphic residues present in exon 2 and exon 3 at positions that are allelic determinants of a DQB1 I allele.

As used herein an "allelic determinant" refers to a polymorphic site where the presence of variation results in variation in the HLA antigen.

The term "target region" refers to a region of a nucleic acid, in the current invention, an HLA gene, that is to be analyzed for the presence of polymorphic sites.

By "oligonucleotide" is meant a single-stranded nucleotide polymer made of more than 2 nucleotide subunits covalently joined together. An oligonucleotide primer as used herein is typically between about 10 and 100 nucleotides in length, usually from 20 to 60 nucleotides in length. The sugar groups of the nucleotide subunits may be ribose, deoxyribose or modified derivatives thereof such as o-methyl ribose. The nucleotide subunits of an oligonucleotide may be joined by phosphodiester linkages, phosphorothioate linkages, methyl phosphonate linkages or by other linkages, including but not limited to rare or non-naturally-occurring linkages, that do not prevent hybridization of the oligonucleotide. Furthermore, an oligonucleotide may have uncommon nucleotides or non-nucleotide moieties. An oligonucleotide as defined herein is a nucleic acid, preferably DNA, but may be RNA or have a combination of ribo- and deoxyribonucleotides covalently linked. Oligonucleotides of a defined sequence may be produced by techniques known to those of ordinary skill in the art, such as by chemical or biochemical synthesis, and by in vitro or in vivo expression from recombinant nucleic acid molecules.

The term "primer" refers to an oligonucleotide that acts as a point of initiation of DNA synthesis under conditions in which synthesis of a primer extension product complementary to a nucleic acid strand is induced in an appropriate buffer and at a suitable temperature. A primer is preferably a single-stranded oligodeoxyribonucleotide. In the current invention, a primer includes an "HLA-binding region" or HLA-hybridizing region" exactly or substantially complementary to the HLA sequence of interest. This region of the primer is typically about 15 to about 25, 30, 35 or 40 nucleotides in length.

As used herein, an "adapter region" of a primer refers to the region of a primer sequence at the 5' end that is universal to the HLA amplicons obtained in accordance with the procedures described herein and provides sequences that anneal to an oligonucleotide present on a microparticle or other solid surface for emulsion PCR. The "adapter region" can further serve as a site to which a sequencing primer binds. The adapter region is typically from 15 to 30 nucleotides in length.

The terms "individual identifier tag", "barcode", "identification tag", "multiplex identification tag", "molecular identification tag" or "MID" are used interchangeably herein to refer to a nucleotide sequence present in a primer that serves as a marker of the DNA obtained from a particular subject.

As used herein, the terms "nucleic acid," "polynucleotide" and "oligonucleotide" refer to primers and oligomer fragments. The terms are not limited by length and are generic to linear polymers of polydeoxyribonucleotides (containing 2-deoxy-D-ribose), polyribonucleotides (containing D-ribose), and any other N-glycoside of a purine or pyrimidine base, or modified purine or pyrimidine bases. These terms include double- and single-stranded DNA, as well as double- and single-stranded RNA.

A nucleic acid, polynucleotide or oligonucleotide can comprise phosphodiester linkages or modified linkages including, but not limited to phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages.

A nucleic acid, polynucleotide or oligonucleotide can comprise the five biologically occurring bases (adenine, guanine, thymine, cytosine and uracil) and/or bases other than the five biologically occurring bases. These bases may serve a number of purposes, e.g., to stabilize or destabilize hybridization; to promote or inhibit probe degradation; or as attachment points for detectable moieties or quencher moieties. For example, a polynucleotide of the invention can contain one or more modified, non-standard, or derivatized base moieties, including, but not limited to, N6-methyl-adenine, N6-tert-butylbenzyl-adenine, imidazole, substituted imidazoles, 5-fluorouracil, 5 bromouracil, 5-chlorouracil, 5-iodouracil, hypoxanthine, xanthine, 4-acetylcytosine, 5 (carboxyhydroxymethyl)uracil, 5 carboxymethylaminomethyl-2-thiouridine, 5 carboxymethylaminomethyluracil, dihydrouracil, beta-D-galactosylqueosine, inosine, N6 isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2 thiocytosine, 5-methyl-2-thiouracil, 2-thiouracil, 4-thiouracil, 5-methyluracil, uracil-5-oxyacetic acidmethylester, 3-(3-amino-3-N-2-carboxypropyl) uracil, (acp3)w, 2,6-diaminopurine, and 5-propynyl pyrimidine. Other examples of modified, non-standard, or derivatized base moieties may be found in U.S. Patent Nos. 6,001,611; 5,955,589; 5,844,106; 5,789,562; 5,750,343; 5,728,525; and 5,679,785. Furthermore, a nucleic acid, polynucleotide or oligonucleotide can comprise one or more modified sugar moieties including, but not limited to, arabinose, 2-fluoroarabinose, xylulose, and a hexose.

The term "amplification conditions" refers to conditions in an amplification reaction (e.g., a PCR amplification) that allow for hybridization of an extendable polynucleotide (e.g., a primer) with a target nucleotide, and the template-dependent extension of the extendable polynucleotide. As used herein, "amplification conditions" or conditions sufficient for amplifying a target nucleic acid are well known in the art. See, e.g., PCR Primer: A Laboratory Manual, by Dieffenbach and Dveksler, eds., 2003, Cold Spring Harbor Press; and PCR Protocols, Bartlett and Stirling, eds., 2003, Humana Press.

The term "amplification" as used here in the context of a nucleic acid amplification reaction refers to a reaction that increases the copies of a nucleic acid template, *e.g.*, the target nucleic acid sequence.

### Introduction

The current invention provides methods of HLA genotyping based the discovery that a multiplex, parallel clonal sequencing analysis can be used to genotype at least 3, typically at least 6, and preferably at least 8 HLA loci in multiple individuals at the same time. Next-generation sequencing methods clonally propagate in parallel millions of single DNA molecules which are then also sequenced in parallel. Recently, the read lengths obtainable by one such next-generation sequencing method (454 Life Sciences, Inc.) have increased to > 250 nucleotides. These clonal read lengths make possible setting the phase of the linked polymorphisms within an exon and thus the unambiguous determination of the sequence of each HLA allele. In the current invention, the system is sufficiently high throughput to enable a complete, 8-locus HLA typing for multiple individuals, *e.g.*, 24 or 48 subjects, in a single sequencing run using a pyrosequencing platform as described herein.

The highly multiplexed amplicon sequencing of the invention employs sample-specific internal sequence tags (barcode tags or MIDs) in the primers that allow pooling of samples yet maintain the ability to assign sequences to a specific individual. In the current invention, the HLA genotypes for at least eight loci (HLA-A, B, C, DRB1, DQA1, DQB1, DPA1, DPB1), as well as for DRB3,4, and 5 can be obtained from the data generated by sequencing. This HLA sequencing system can also detect chimeric mixtures, *e.g.*, the detection of the rare non-transmitted maternal allele present in the blood of SCID patients.

### HLA genes

The human leukocyte antigen system (HLA) complex spans approximately 3.5 million base pairs on the short arm of chromosome 6. The major regions are the class I and class II regions. The major Class I antigens are HLA-A, HLA-B, and HLA-C and the major Class II antigens are HLA-DP, HLA-DQ and HLA-DR. The HLA-DP, HLA-DQ and HLA-DR loci encode the a and β chains of the HLA-DR, DP and DQ antigens. The HLA genes are among the most polymorphic genes. Polymorphisms that are expressed in the HLA antigen (and therefore of great interest for typing for transplantation) are localized primarily in exon 2 for the class II genes and exons 2 and 3 for the class I genes.

In the current invention, the genotype of an HLA gene as described herein refers to determining the polymorphisms present in that HLA gene. For HLA-A, the polymorphisms present in exon 2 and exon 3 are determined by sequencing amplicons generated by PCR from an individual. In typical embodiments, the sequence of exon 4 is also determined. Exon 2, exon 3, and exon 4, or regions thereof that comprise the allelic determinants, are each amplified in individual PCR reactions to obtain amplicons. Similarly, amplicons are obtained for exon 2 and exon 3, and in some embodiments, exon 4, for the HLA-B and HLA-C alleles for an individual. For genotyping HLA class II alleles, amplicons are obtained for exon 2 of DRB1, DPB1, DPA1, DQA1 and exons 2 and 3 of DQB1. Each exon can be sequenced completely by sequencing both strands with sufficient overlap between the reads from either end that specific HLA alleles can be unambiguously assigned.

Each sample from an individual is amplified at each exon individually using primers that target the exon of interest, or the polymorphic region of the exon of interest, for amplification. The primers employed in the amplification reaction include additional sequences: adapter sequences for emulsion PCR and an identifying sequence that serves as a marker for the DNA from a single individual.

### Amplification primers

The invention employs amplification primers that amplify the exons of interest of the HLA genes. Typically, the primers are designed to ensure that the entire polymorphic portion of the exon is obtained.

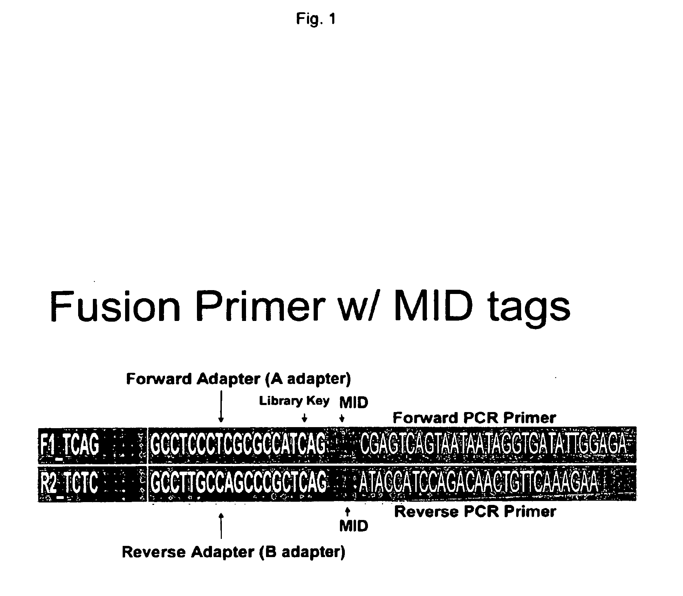

In the current invention, primer sequences for the multiplex amplification of the invention are designed to include sequences that can be used to facilitate the clonal sequencing and the analysis. The amplification primers of the invention (also referred to herein as "fusion primers") therefore include the following components: an adaptor, a unique identification tag and a sequence that hybridizes to an HLA gene of interest to use in an amplification reaction to obtain an HLA amplicon. Figure 1 provides a schematic showing a fusion primer of the invention.

The adaptor portions of the primer sequences are present at the 5' end of the amplicon fusion primers. The adapter regions comprise sequences that serve as the site of annealing of primers for the sequencing reaction and also correspond to sequences present on beads, or a solid surface, so that the amplicon can be annealed to the surface for emulsion PCR. The forward primer for amplifying an HLA exon includes an adapter sequence at the 5' end, referred to here as the adapter region A. The reverse primer comprises a region that contains an adapter sequence at the 5' end, referred to here as adapter region B. As noted, the sequences present in the adaptor region and their complements allow for annealing of the amplicons to beads for emulsion PCR. Optionally, the adaptor may further include a unique discriminating key sequence comprised of a non-repeating nucleotide sequence (i.e., ACGT, CAGT, etc.). This key sequence is typically incorporated to distinguish the amplicons for HLA genotyping from control sequences that are included in the reaction. Such sequences are described, *e.g.*, in WO/2004/069849 and WO 2005/073410 Additional guidance for configuring adapter primers is provided, *e.g.*, in WO/2006/110855.

In some embodiments, the adapter sequences for use in the invention are the primer A and primer B sequences for the 454 GS-FLX 454 sequencing system (Roche Diagnostics). The primer A sequence is 5' GCCTCCCTCGCGCCA 3' (SEQ ID NO: 1). The primer B sequence is 5' GCCTTGCCAGCCCGC 3' (SEQ ID NO: 2). As noted above, the primers typically contain additional "key" sequences that provide identifying sequencing to distinguish the amplicons from control sequences.

PCR primers for use in the HLA genotyping methods of the invention further comprise individual identifier tags. These individual identifier tags are used to mark the HLA amplicons from each individual who is being tested. The HLA sequences of interest are amplified from a nucleic acid sample from a subject to be genotyped. As explained above, the HLA exons, or regions of the exons, comprising the polymorphisms that act as allelic determinants are individually amplified. The amplicons obtained from the subject are marked with the same identification tag. The tag is included in the fusion primers that are used to amplify each amplicon for that subject. Accordingly, the identification tags are also sequenced in the sequencing reaction. The ID tags are present in the fusion primers used to obtain the HLA amplicons between the adapter region and the HLA priming region of the fusion primer.

Identification tags may vary in length. Typically, the tag is at least 4 or 5 nucleotides in length. In some applications, it may be desirable to have longer identification sequences, *e.g.*, 6, 8, or 10 or more nucleotides in length. The use of such sequences is well known in the art. (*see*, *eg..*, Thomas, et al. Nat. Med., 12:852-855, 2006; Parameswaran et al,. Nucl. Acids Res., 35:e130, 2007; Hofmann et al., Nucl. Acids Res. 35:e91, 2007). In most embodiments of this invention, the identification tag is from 4 to 10 nucleotides in length.

Individual identifier sequences can be designed taking into account certain parameters. For example, in designing a 4-residue ID tag, it is desirable to choose 4 bases that take into account the flow cycle of the nucleotides in the sequencing reaction. For example, if the nucleotides are added in the order T, A, C, and G, it is typically desirable to design the tag sequence such that a residue that is positive is followed by a residue that would be negative. Accordingly, in this example, if a tag sequence begins with an "A" residue such that the nucleotide incorporated in the sequencing reaction is T, the second residue in the tag sequence would be a nucleotide such that A would not be incorporated. In addition, it is desirable to avoid forming homopolymers, either within the tag sequence or through creating them based on the last base of the adapter region or the first base of the HLA-specific region of the fusion primer.

The HLA priming region (also referred to herein as HLA binding region, or HLA hybridizing region) of the fusion primers is the region of the primer that hybridizes to the HLA sequence of interest to amplify the desired exon (or in some embodiments, region of the exon). Typically, the HLA region of the fusion primer hybridizes to intronic sequence adjacent to the exon to be amplified in order to obtain the entire exon sequence. The HLA sequences are preferably selected to selectively amplify the HLA exon of interest, although in some embodiments, a primer pair may also amplify a highly similar region of a related HLA gene. For example, the primers for exon 2 of DRB 1 described in the example section below also amplify the DRB3, DRB4, and DRB5 loci. The primers are selected such that the exon is amplified with sufficient specificity to allow unambiguous determination of the HLA genotype from the sequence.

Sequences of HLA genes and alleles are known and available through various databases, including GenBank and other gene databases and have been published (see e.g., Mason and Parham (1998) Tissue Antigens 51: 417-66, listing HLA-A, HLA-B, and HLA-C alleles; Marsh et al. (1992) Hum. Immunol. 35:1, listing HLA Class II alleles--DRA, DRB, DQA1, DQB1, DPA1, and DPB1).

The PCR primers can be designed based on principles known in the art. Strategies for primer design may be found throughout the scientific literature, for example, in Rubin, E. and A. A. Levy, Nucleic Acids Res, 1996.24 (18): p. 3538-45; and Buck et al., Biotechniques, 1999.27 (3): p. 528-36. For example, the HLA-specific region of the primer is typically about 20 nucleotides or greater, *e.g.*, 20 to 35 nucleotides in length. Other parameters that are considered are G/C content, design considerations to avoid internal secondary structure, and prevent the formation of primer dimers, as well as melting temperatures (Tₘ).

Examples of primers for use in this invention are provided in Table 1. In Table 1, the forward primers have the 454 sequencing system "A" primer sequence at the 5' end, followed by a four nucleotide key (TCAG), which together comprise the adapter region; followed by the identifier tag (4 nucleotides, unless otherwise noted); which is then followed by the region that hybridizes to the HLA gene indicated. The reverse primers have the 454 sequencing system "B" primer sequence at the 5' end followed by the four nucleotide key TCAG", which together comprise the adapter region, followed by the identifier tag region, followed by the HLA-specific region.

A primer used in the methods of the invention may comprise an HLA-hybridizing region of a primer set forth in Table 1. In other embodiments, such a primer may comprise a portion that is substantially identical to the sequence of an HLA hybridizing region set forth in Table 1. Thus, for example, a primer of the invention may comprise at least 10, 15, or 20 or more contiguous nucleotides of an HLA hybridizing region of a primer set forth in Table 1.

The HLA amplifications for each subject to be HLA genotyped are performed separately. The amplicons from the individual subject are then pooled for subsequent emulsion PCR and sequence analysis.

The template nucleic acid used to amplify the HLA amplicon of interest is typically from genomic DNA isolated from a subject to be genotyped. In the current method, more than one subject is HLA genotyped in parallel reactions. In the current invention, at least 12 subjects, and typically at least 16, 20, 24, 30, 36, or 48 subjects are HLA genotyped.

The HLA amplicons may be obtained using any type of amplification reaction. In the current invention, multiplex amplicons are typically made by PCR using primer pairs as described herein. It is typically desirable to use a polymerase with a low error rate, *e.g.*, such as a high-fidelity Taq polymerase (Roche Diagnostics).

The PCR conditions can be optimized to determine suitable conditions for obtaining HLA amplicons from a subject. Each HLA amplicon may be individually amplified in separate PCR reactions. In some embodiments, the HLA amplicons for a subject may be obtained in one or more multiplex reactions that comprise primer pairs to amplify individual amplicons

### Emulsion PCR

The HLA amplicons are attached to beads and subject to emulsion PCR. Emulsion PCR is known in the art (see, e.g., WO/2004/060849, WO 2005/073410, U.S. Patent Application Publication No. 20050130173, WO/2007/086935 and WO/2008/076842). In emulsion PCR, amplification is performed by attaching a template to be amplified, in the current invention, an HLA amplicon, to a solid support, preferably in the form of a generally spherical bead.

The HLA amplicon is attached to the bead by annealing the amplicon, via the adaptor region, to a primer attached to a bead. Thus, the bead is linked to a large number of a single primer species that is complementary to the HLA amplicon in the adapter portion. The beads are suspended in aqueous reaction mixture and then encapsulated in a water-in-oil emulsion. The emulsion is composed of discrete aqueous phase microdroplets, *e.g.*, approximately 60 to 200 µm in diameter, enclosed by a thermostable oil phase. Oil is added and emulsion droplets are formed such that on average, the emulsion comprises only one target nucleic acid and one bead. Each microdroplet contains, preferably, amplification reaction solution (i.e., the reagents necessary for nucleic acid amplification, such as polymerase, salts, and appropriate primers, *e.g.*, corresponding to the adaptor region).

In the current invention, emulsion PCR is typically performed with two populations of beads, as the HLA amplicons are sequenced in both directions. In one population of beads, a first primer corresponding to the adapter sequence present on the reverse primer is attached to a bead. In the second population, a second primer corresponding to the adapter sequence present on the forward primer is attached to a bead. Thus, a primer for use in the emulsion amplification reaction typically has the sequence of the adapter region, without additional sequences such as "key" sequences. The emulsion amplification reaction is typically performed asymmetrically. For example, the PCR primers may be present in a 8:1 or 16:1 ratio (i.e., 8 or 16 of one primer to 1 of the second primer) to perform asymmetric PCR.

Following emulsion amplification, the beads that have the singled-stranded HLA amplicon template are isolated, *e.g.*, via a moiety such as a biotin that is present on an amplification primer during the emulsion PCR, and the template is sequenced using DNA sequencing technology that is based on the detection of base incorporation by the release of a pyrophosphate and simultaneous enzymatic nucleotide degradation (described, *e.g.*, in U.S. Patent Nos. 6,274,320, 6,258,568 and 6,210,891).

Clonal amplicons are sequenced using a sequencing primer (*e.g.*, primer A or primer B) and adding four different dNTPs or ddNTPs subjected to a polymerase reaction. As each dNTP or ddNTP is added to the primer extension product, a pyrophosphate molecule is released. Pyrophosphate release can be detected enzymatically, such as, by the generation of light in a luciferase-luciferin reaction. Additionally, a nucleotide degrading enzyme, such as apyrase, can be present during the reaction in order to degrade unincorporated nucleotides (see, e.g., U.S. Patent. No. 6258568.) In other embodiments, the reaction can be carried out in the presence of a sequencing primer, polymerase, a nucleotide degrading enzyme, deoxynucleotide triphosphates, and a pyrophosphate detection system comprising ATP sulfurylase and luciferase (see, e.g., U.S. Pat. No. 6258568).

Once the sequencing data is obtained for the sequence of the individual DNA molecules, the unambiguous exon sequence can be determined by comparing these sequence files to an HLA sequence database for the two HLA alleles The read lengths achieved by the GSFLX system (454 Life Sciences) (avg = 250bp) allow sufficient overlap for this determination of each exon. The assignment of genotypes at each locus based on the exon sequence data files can be performed, *e.g.*, by a software developed by Conexio Genomics. An important aspect of the software is the ability to filter out related sequence reads (pseudogenes and other unwanted HLA genes) that were co-amplified by the primers along with the target sequence.

### Kits

The compositions and reagents described herein can be packaged into kits. A kit of the invention typically comprises multiple primer pairs as described herein that are suitable for amplifying the regions of interest in an HLA allele. The primer pairs comprise a forward primer comprising an adapter region, an individual identification tag and an HLA hybridizing region; and a reverse primer that comprises an adapter region, an individual identification tag, and an HLA hybridizing region. The kits of the invention often comprise primer pairs to amplify amplicons for determining the genotype of multiple subjects for at least HLA-A, HLA-B, and DRB1. Often, a kit of the invention comprises sufficient primer pairs to determine the genotype of HLA-A, HLA-B, HLA-C, DRB1, DQA1, DQB1, DPA1, and DPB1 genes for multiple individuals, *e.g.*, 12 or more individuals.

In some embodiments, a kit can additionally comprise one or more populations of beads that have a primer attached that corresponds to an adapter regions that can be used in emulsion PCR. In some embodiments, a kit can comprise one or more reaction compartments comprising reagents suitable for performing a reaction selected at the discretion of a practitioner. For example, in some embodiments, a kit can comprise one or more reaction compartments comprising one more sequencing reagents.

The various components included in the kit are typically contained in separate containers, however, in some embodiments, one or more of the components can be present in the same container. Additionally, kits can comprise any combination of the compositions and reagents described herein. In some embodiments, kits can comprise additional reagents that may be necessary or optional for performing the disclosed methods. Such reagents include, but are not limited to, buffers, control polynucleotides, and the like.

In this application, the use of the singular includes the plural unless specifically stated otherwise. The section headings used herein are for organizational purposes only and are not to be construed as limiting the subject matter described in any way. While the present teachings are described in conjunction with various examples, it is not intended that the present teachings be limited to such embodiments.

### EXAMPLES

### Multiplex Pyrosequencing

The analysis of multiple HLA loci for multiple samples in a single 454 run is facilitated by the incorporation of molecular ID (MID) tags into the PCR primers. Table 1 shows the sequences of the 454 HLA-specific fusion primers with the adapter sequence (for bead capture) and a 4-base MID tag. Additional sequences are provided that gave a 5-base MID tag.

In an initial experiment 24 cell lines having known HLA genotypes (Table 2) were analyzed. In a subsequent experiment, 48 samples were analyzed.

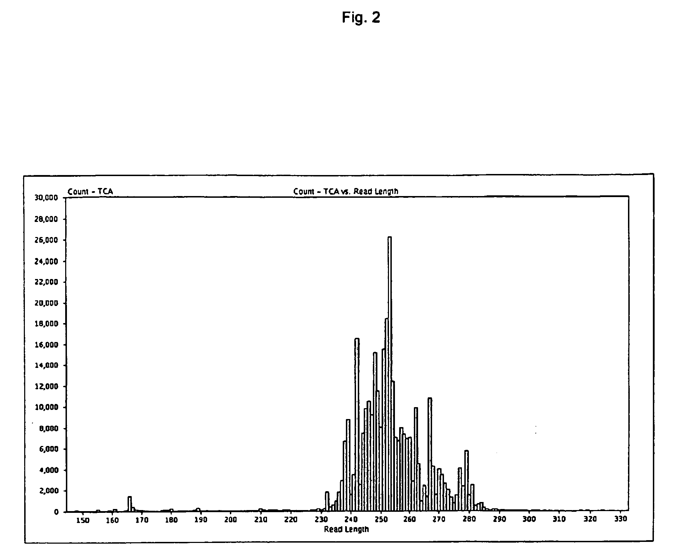

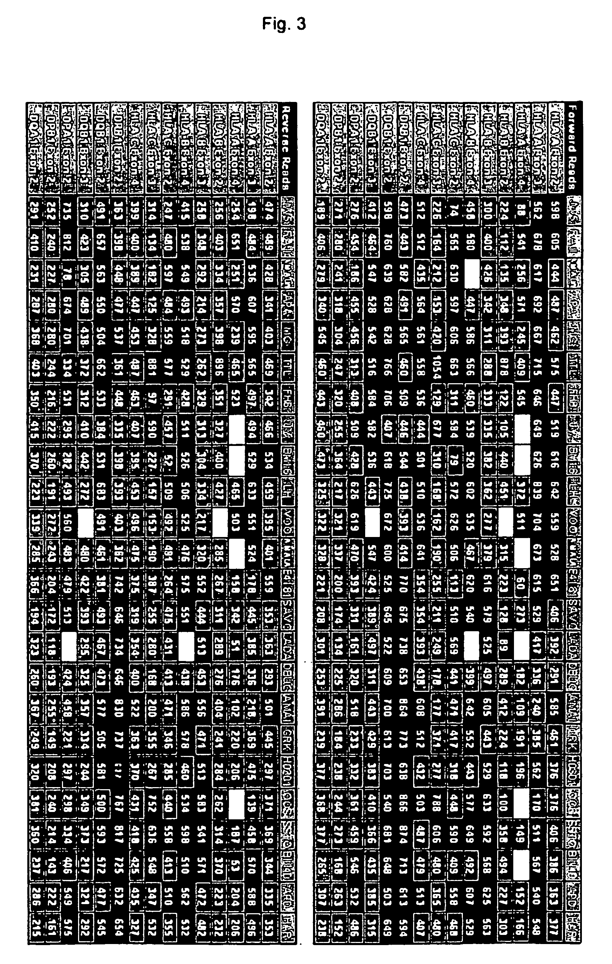

Fifteen primer pairs were designed for the exons 2,3, and 4 of HLA-A, B and C loci, exon 2 of DRB1, DPB1, DPA1, DQA1, and exons 2 and 3 of DQB1. Primers with twelve different MID tags for each target sequence were designed for a total of 180 (15 X 12). The primers for exon 2 of DRB1 also amplify the DRB3, DRB4, and DRB5 loci, genes that are present on specific DRB1 haplotypes. Following amplification of the various samples, the PCR products were quantified by BioAnalyzer analysis , diluted to the appropriate concentration, and pooled for the emulsion PCR. Pyrosequencing runs of 24 and 48 individuals were achieved using 2 or 4 picotitre plate regions, respectively. The distribution of read lengths for all amplicons is shown in Figure 2. The average length was 250 bp. This length is sufficient for the forward and reverse sequence reads to overlap, allowing unambiguous assignment of sequence to each exon and, ultimately, to each allele. The numbers of reads for each exon per individual are shown in Figure 3.

### Genotyping Software

To facilitate genotype assignment from these complex sequence data files, a software program was developed (Conexio Genomics) that compares the forward and reverse sequence reads derived from each exon to an HLA sequence database. The database also contains the sequence of HLA pseudogenes and related genes, allowing the filtering out of sequences generated from pseudogenes or from non classical HLA class I genes (e.g. HLA-E, F, G, and H).

Twenty four cell-line derived DNA samples of known HLA type, based on probe hybridization HLA typing and Sanger sequencing, were sequenced at all 8 loci (HLA-A, -B,-C, -DRB1, -DQA1, -DQB1, DPA1, DPB1). Exon 2 sequences of DRB3, DRB4, and DRB5 were also identified in the amplicons generated by the DRB primer pair. Subsequently, a run of 48 samples (24 cell line DNAs and 24 DNAs extracted from blood samples) were sequenced at the same loci and genotype assignments were generated from the sequence data by Conexio ATF software. The concordance of software genotype calls and previously determined HLA types was 99.4%.

### Analysis of chimeric mixtures (rare variant detection)

The very high number of sequence reads (n=300-350K) generated in a typical GSFLX run makes possible the detection of rare variant sequences present in the sample. To estimate the sensitivity to detect such sequences, we prepared mixtures of PCR products for exons 2 and 3 of HLA-A and HLA-B and exon 2 of DRB from two HLA homozygote samples in various proportions (1/1, 1/10, 1/100, 1/1000). The rare variant present in mixtures of 1/100 could be detected reproducibly.

The blood of certain individuals is chimeric, with residual maternal cells present at very low levels in the child's circulation or rare fetal cells maintained in the mother's circulation (ref.) SCID patients often retain maternal cells at a very low level. When such patients are recipients of hemapoetic stem cell transplant, characterizing the level of this potential chimerism is clinically important. To mimic the SCIDS situation, in which maternal cells may be present in a child, we prepared mixtures of two heterozygous samples, which shared one allele, in various proportions. In this experiment, the rare variant could be detected.

Two SCIDS patients, who were recipients of HST transplants were also analyzed, along with their parents. In each case, the presence of the non-transmitted material allele could be detected.

Clonal sequencing, the analysis of amplicons generated from individual DNA molecules amplified in turn from HLA exons allows the unambiguous exon sequence determination and, by comparing these sequence files to an HLA sequence database, determination of the two HLA alleles.The read lengths achieved by the GSFLX system (454 Life Sciences) (avg = 250bp) allow sufficient overlap for this determination of each exon. In the present examples, the assignment of genotypes at each locus based on the exon sequence data files was performed by a software (ATF) developed by Conexio Genomics. An important aspect of the software is the ability to filter out related sequence reads (pseudogenes and other unwanted HLA genes) that were co-amplified by the primers along with the target sequence. The software also filters out very rare sequence reads that may have been generated by an error in the initial PCR amplification of the target sequence from genomic DNA, errors in the emulsion PCR, or pyrosequencing errors. One well-documented category of pyrosequencing errors is in the length determination of homopolymer tracts. For example, we have observed, rare sequence reads containing a run of Gs when most sequence reads contained the correct run of - Gs.

The cost of a single GSFLX run is considerable. To make this system cost-effective for high resolution clinical HLA typing, multiple samples are analyzed at multiple loci in a single run. The use of MID tags and multiple regions of the picotitre plate makes running 24 or 48 samples analyzed at 8 loci possible, as described in these examples.

It is the very large number of sequence reads generated in parallel that allows this multiplex analysis of multiple individuals at multiple loci It also provides the capacity to detect rare variants sequences. In mixtures of PCR products from two different genomic DNA samples, HLA exon sequences present at 1/100 were reliably detected. Related but unwanted sequences as well as rare sequences containing errors can also be filtered out. (Most HLA alleles differ from one another by multiple polymorphisms while the sequences containing errors typically differ from the correct sequence by only one nucleotide.)

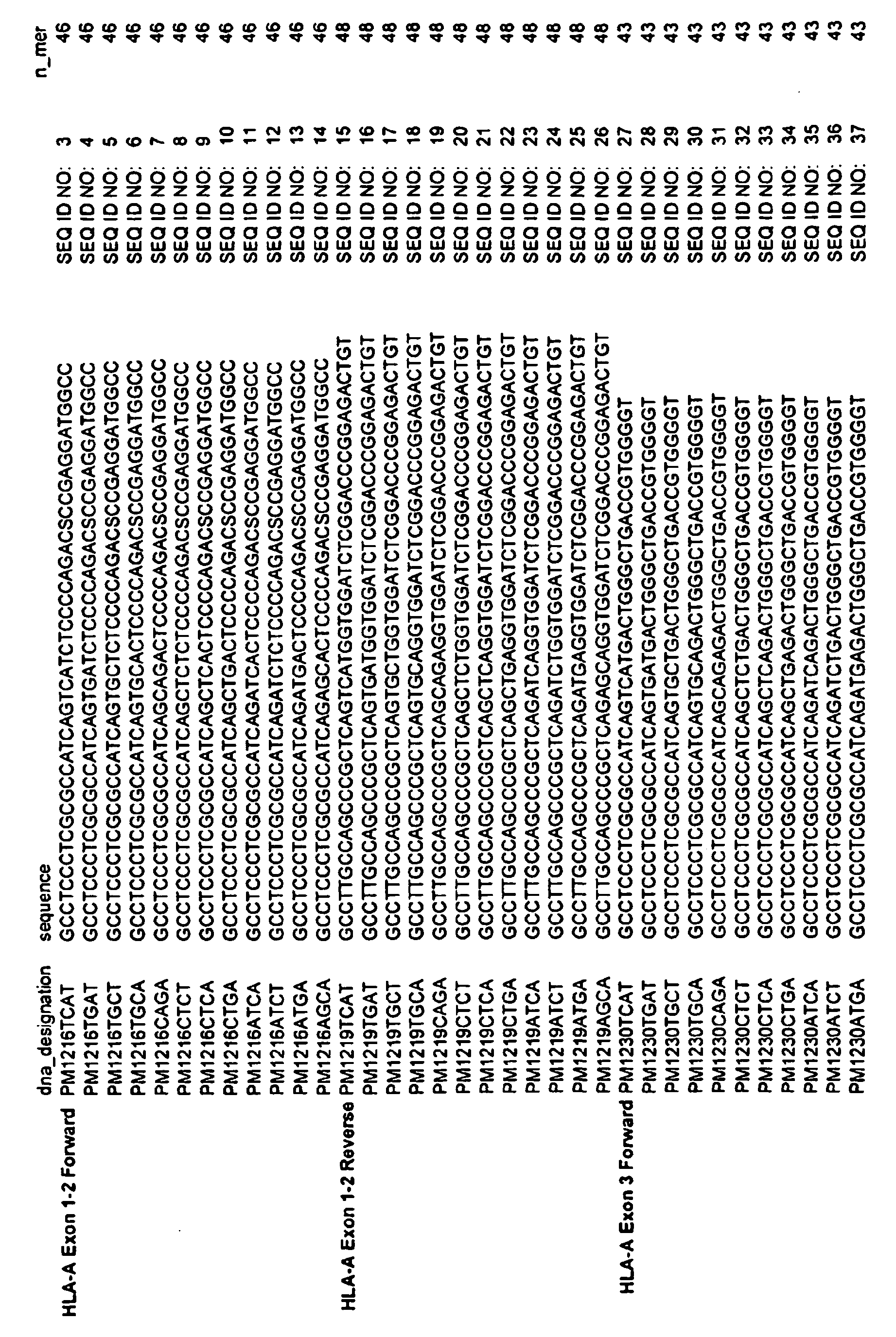

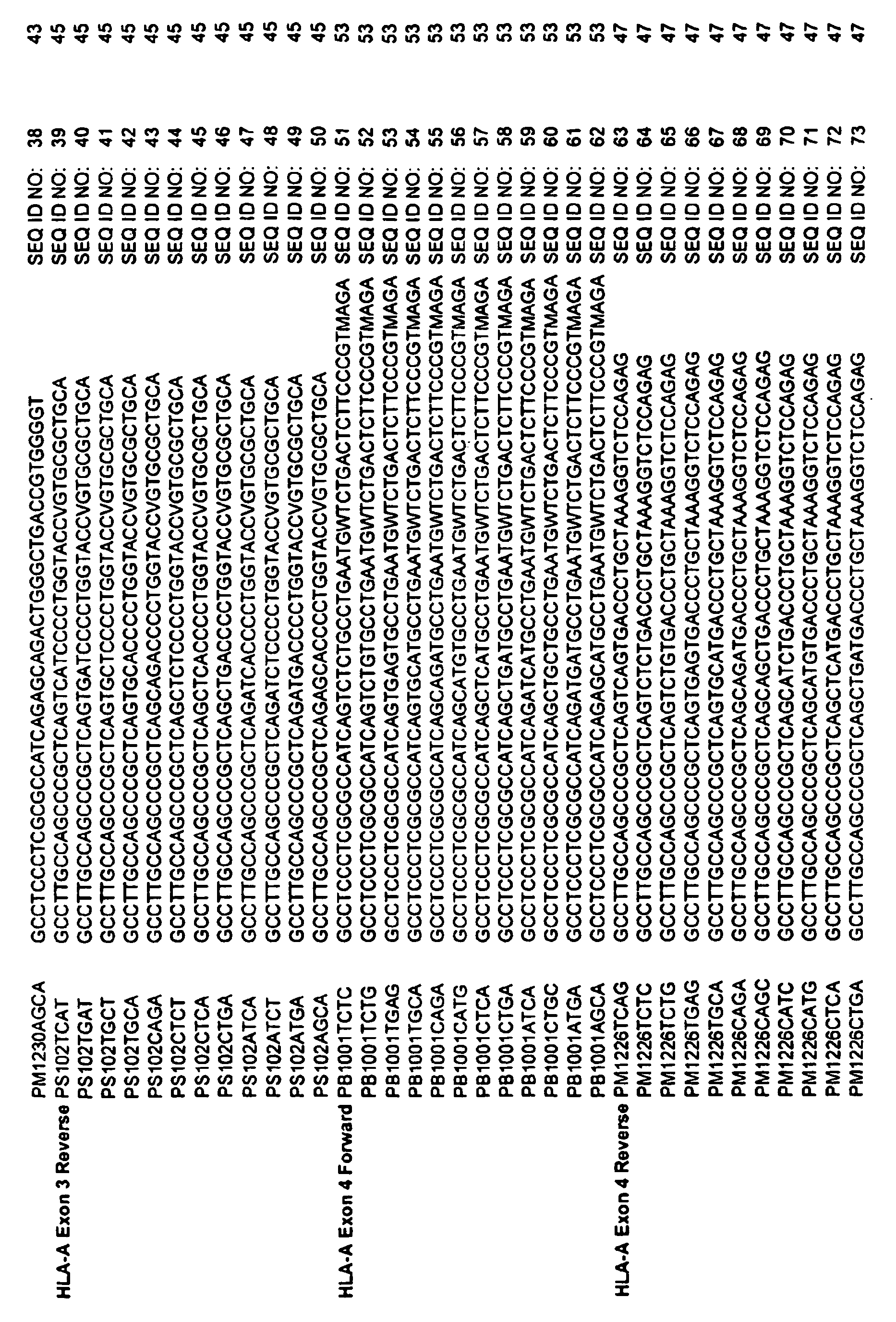

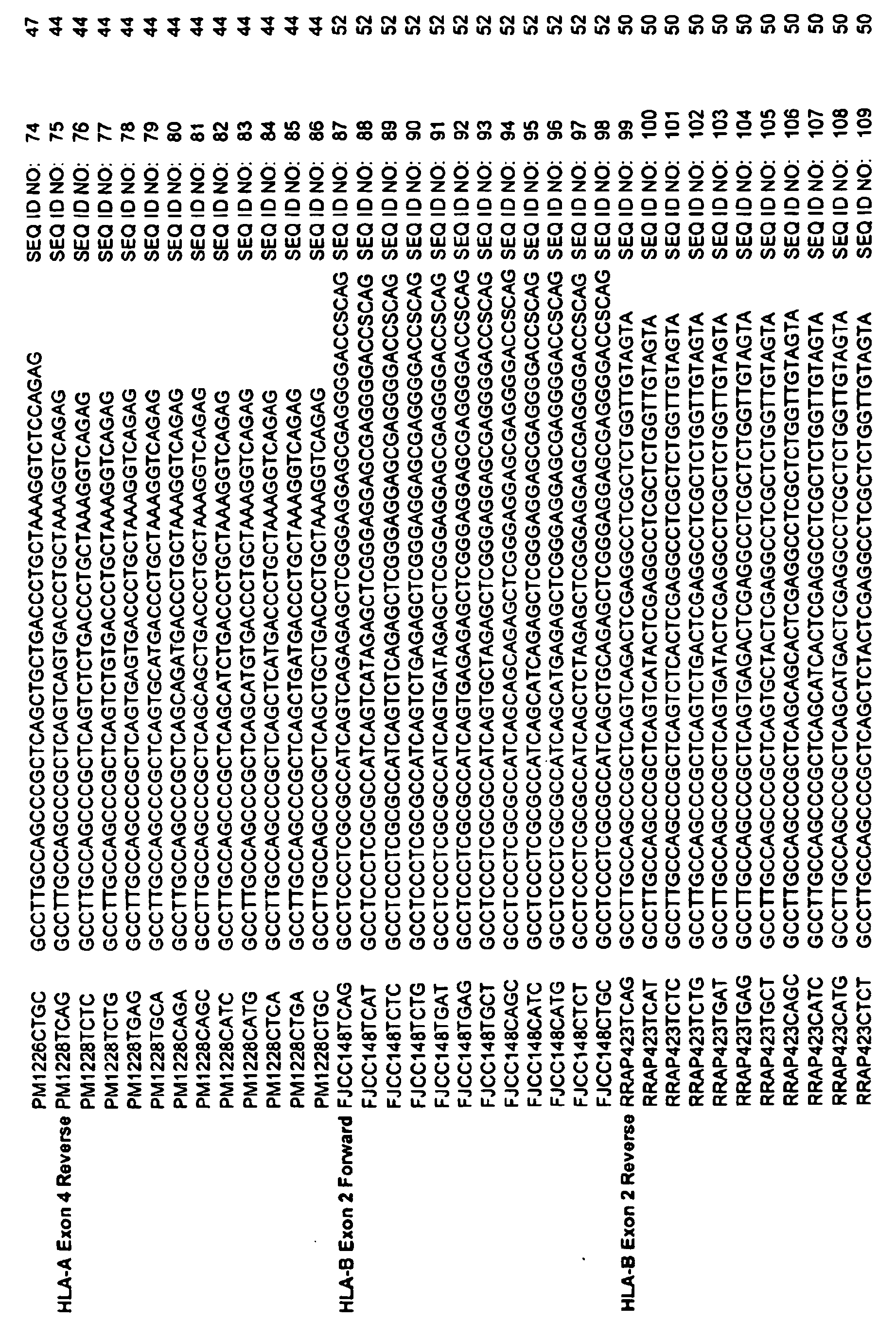

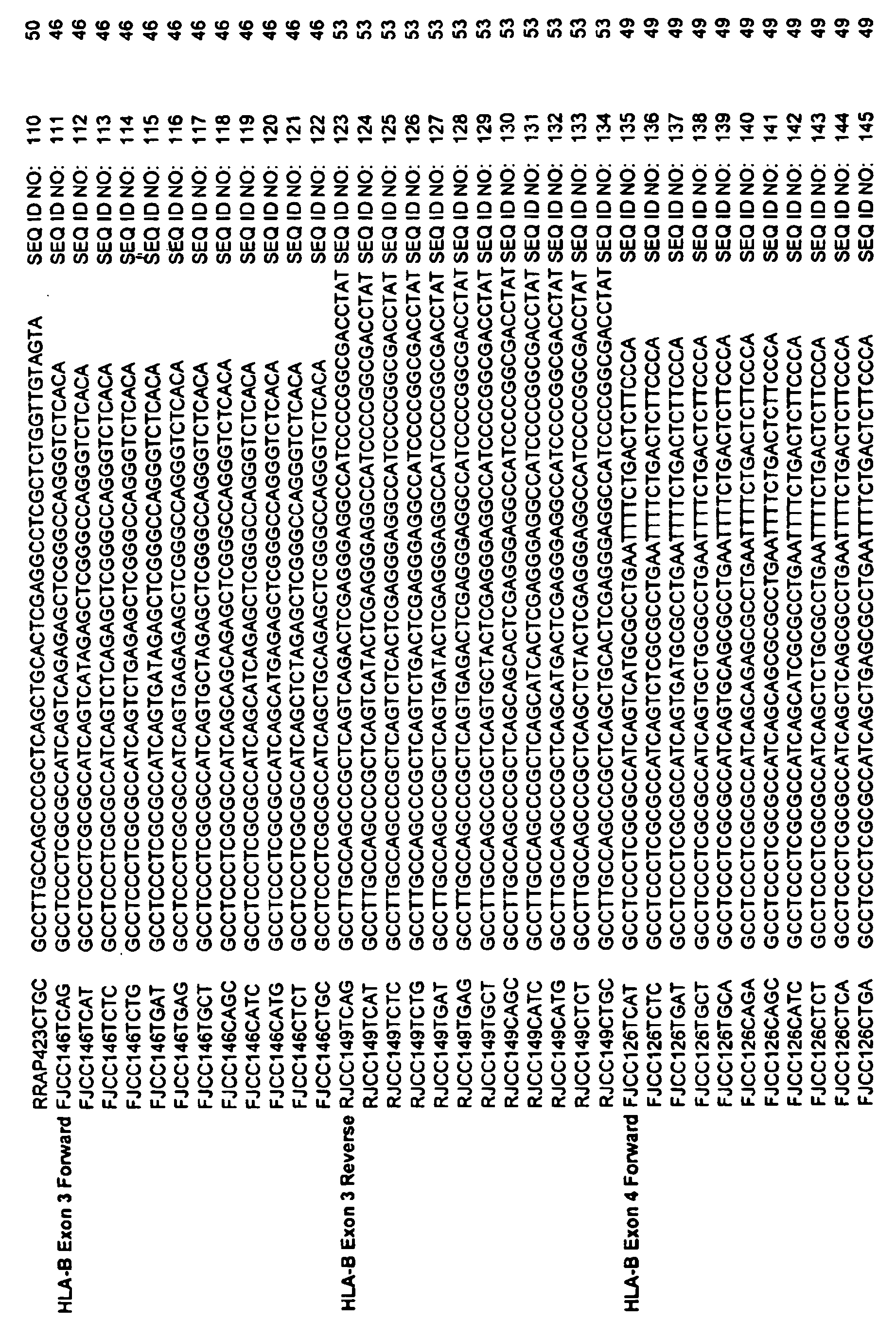

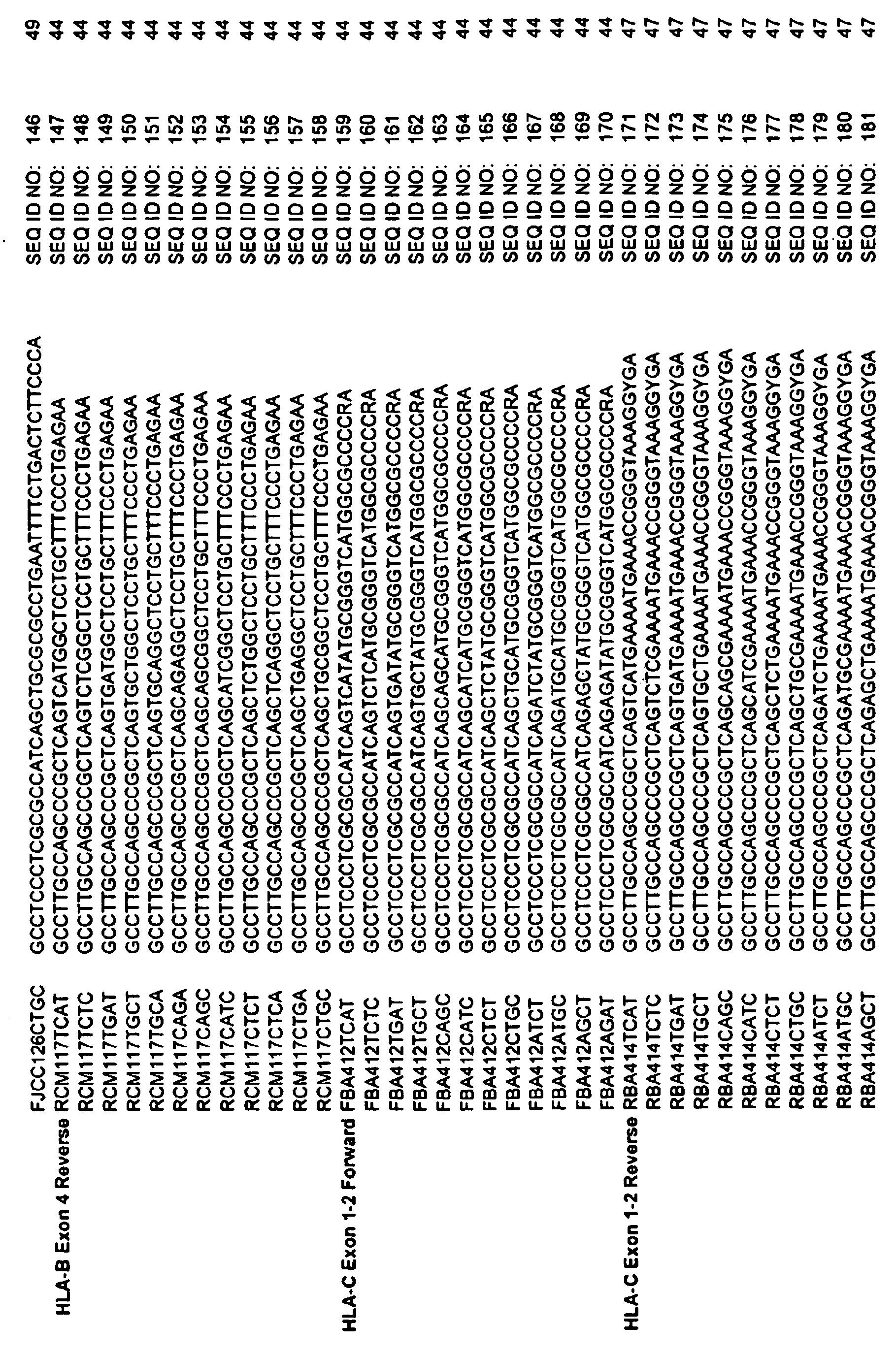

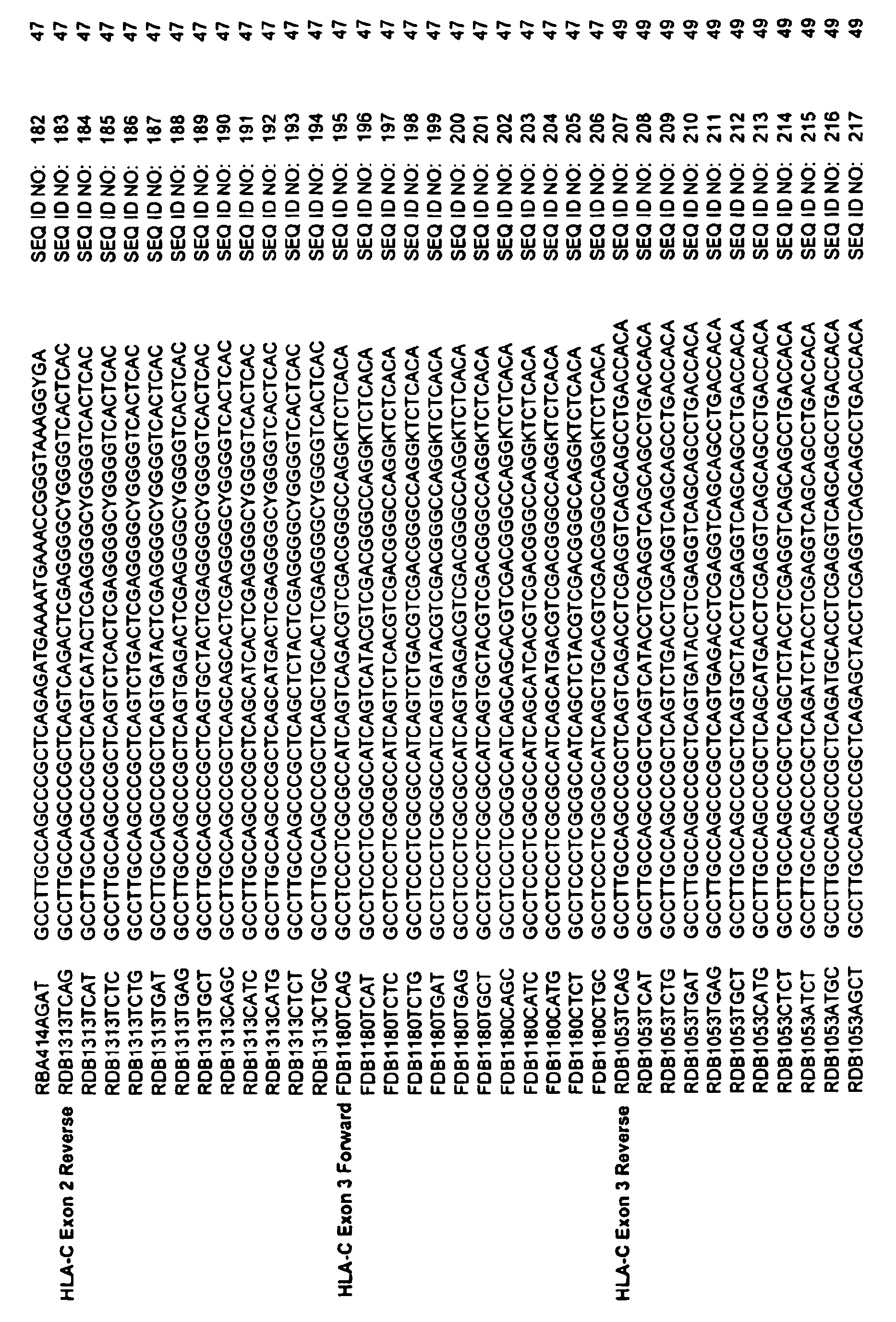

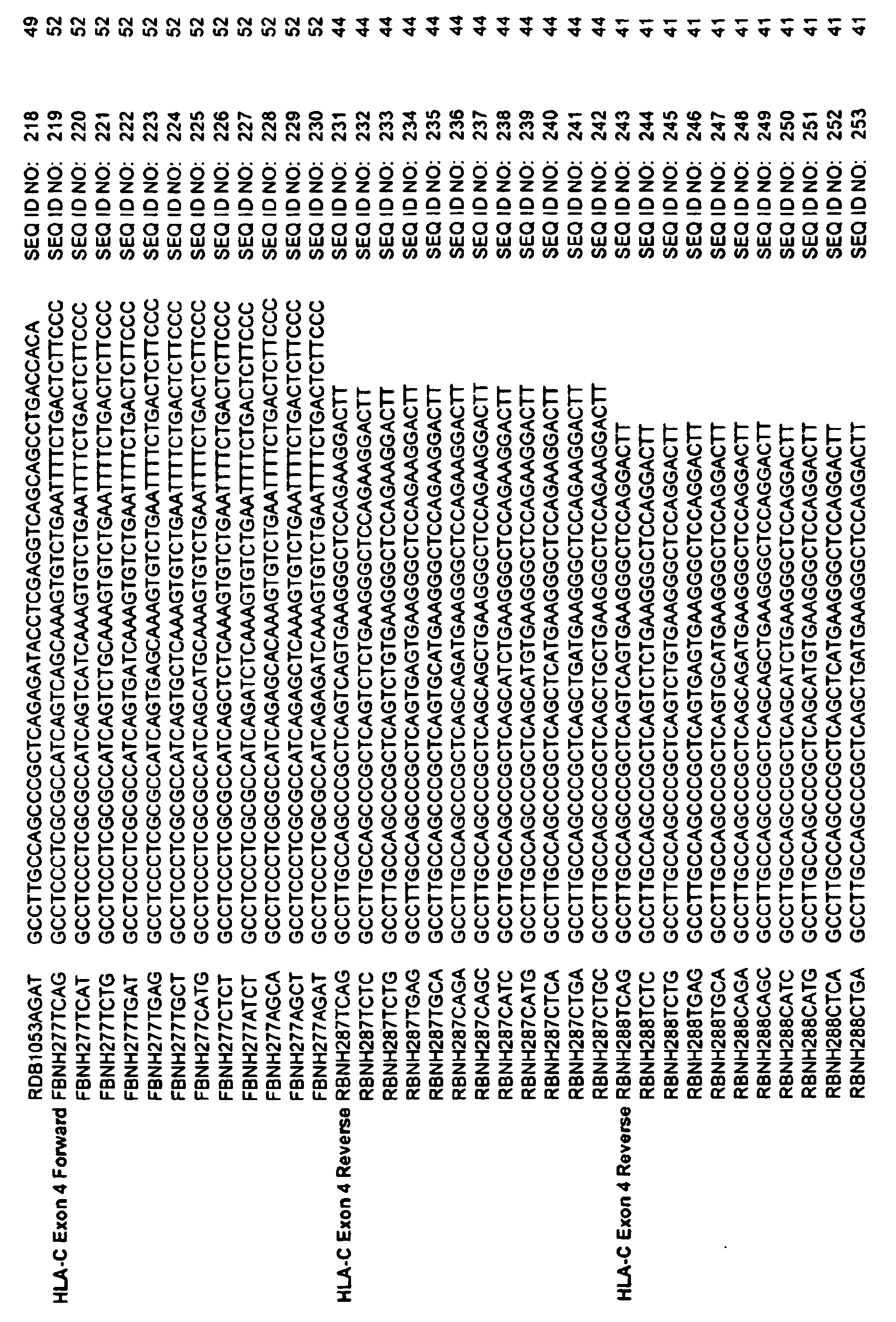

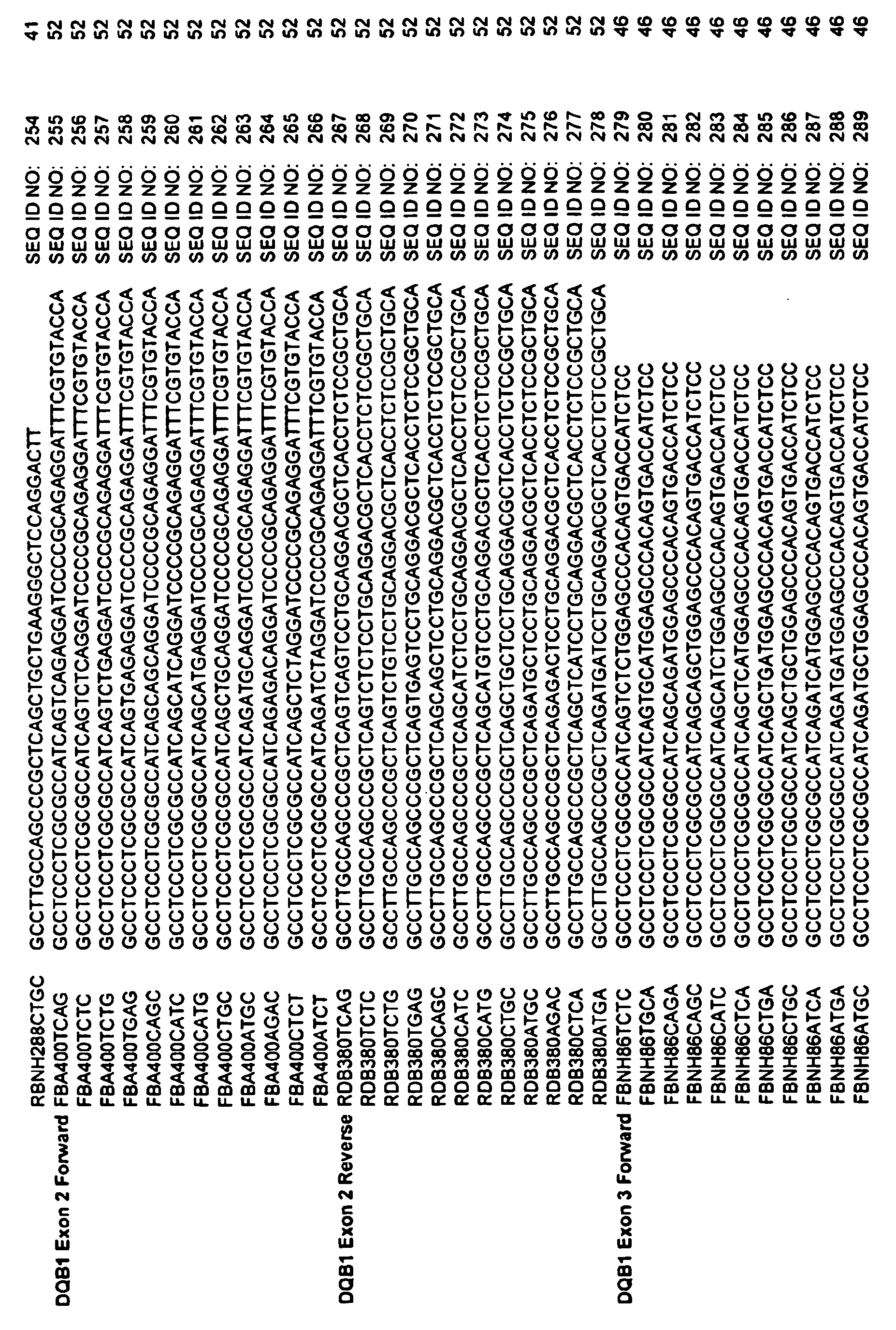

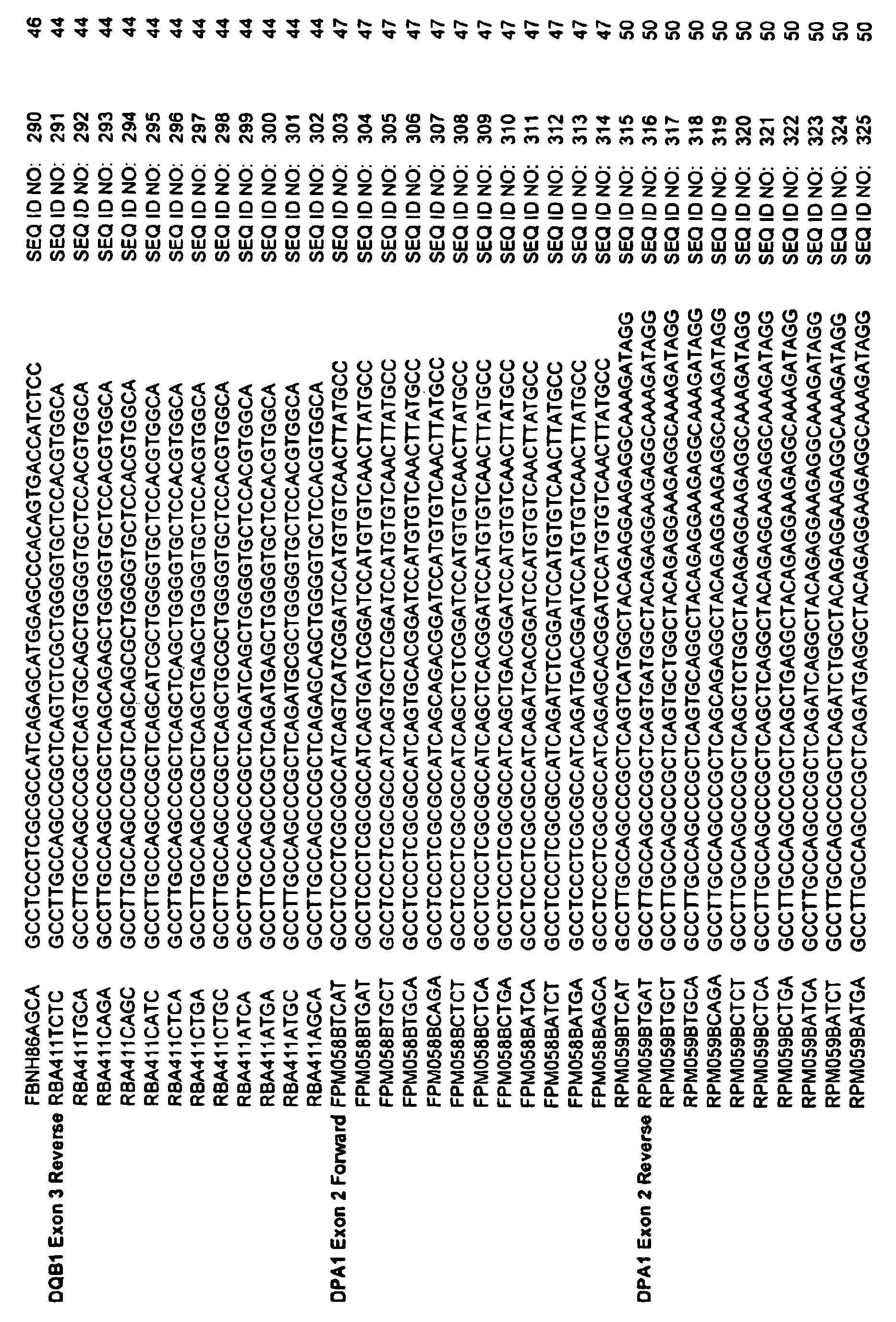

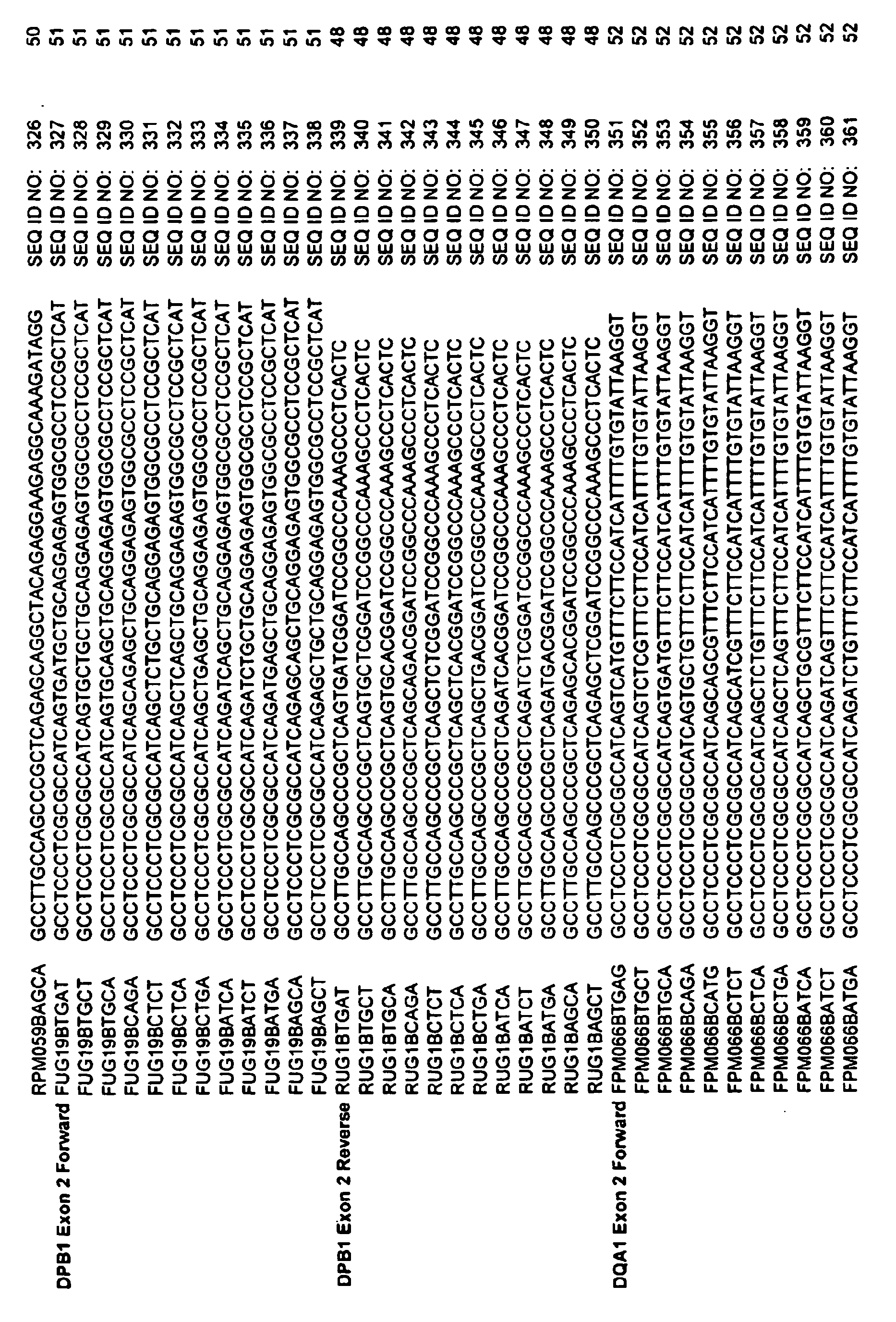

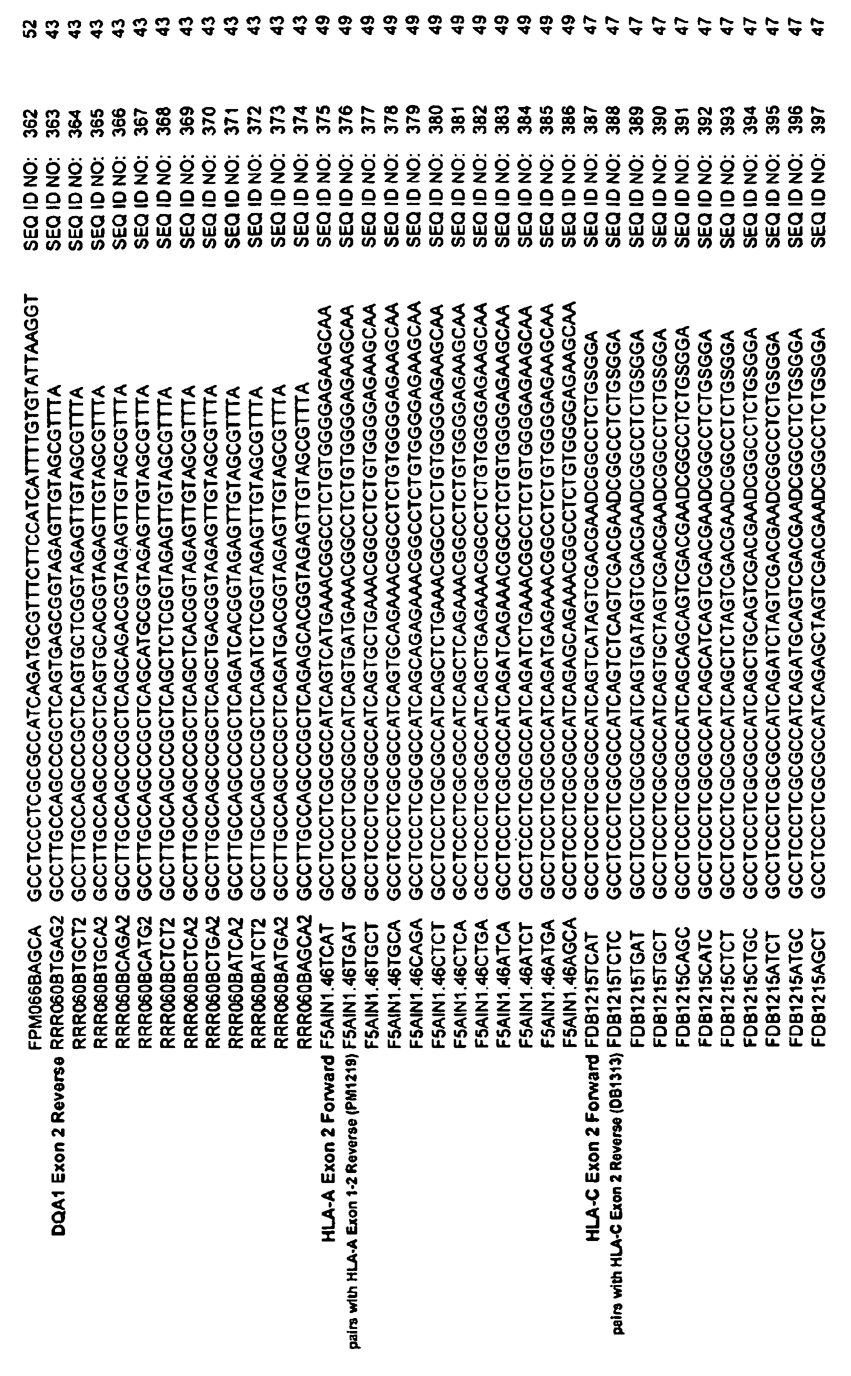

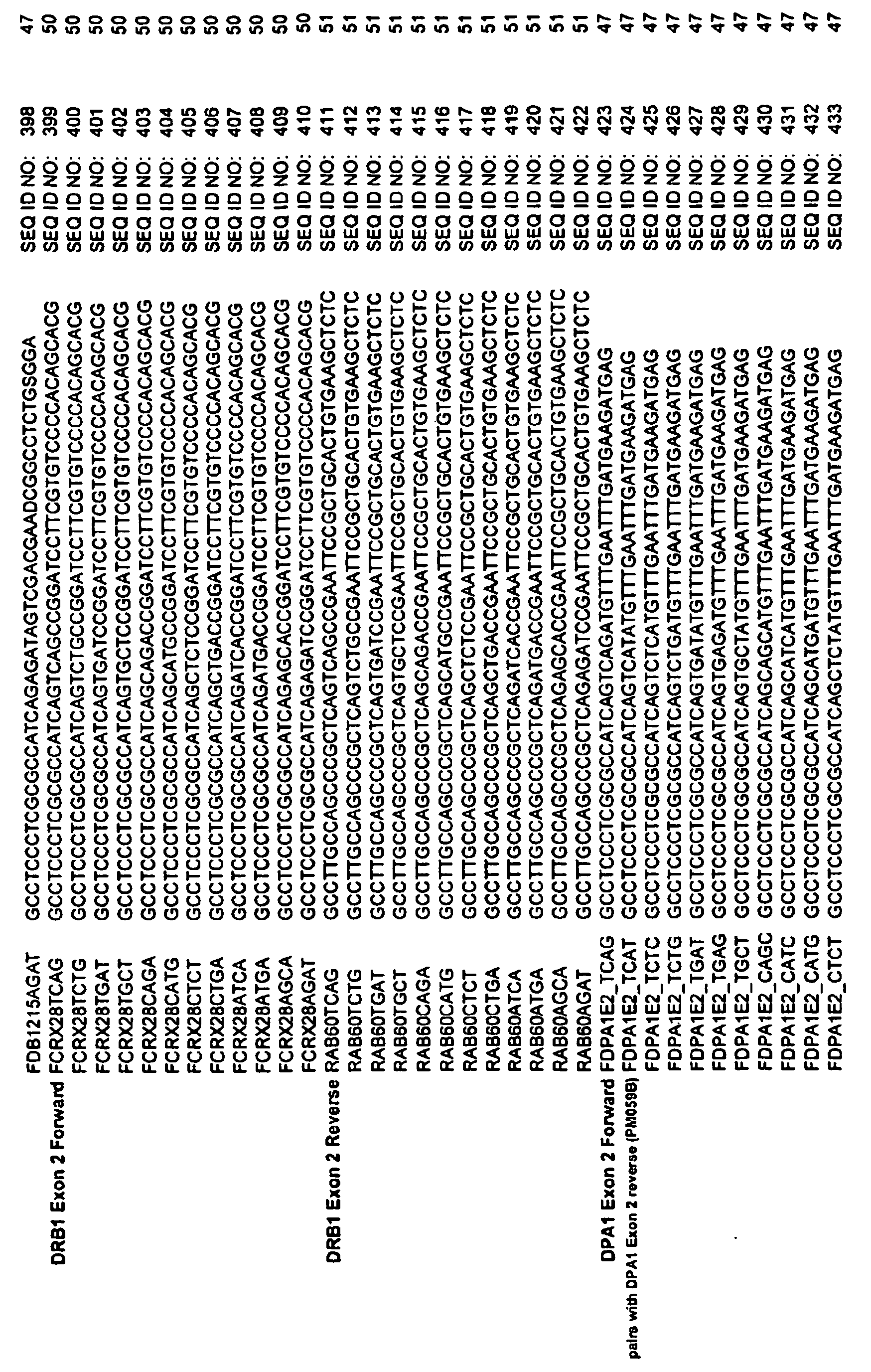

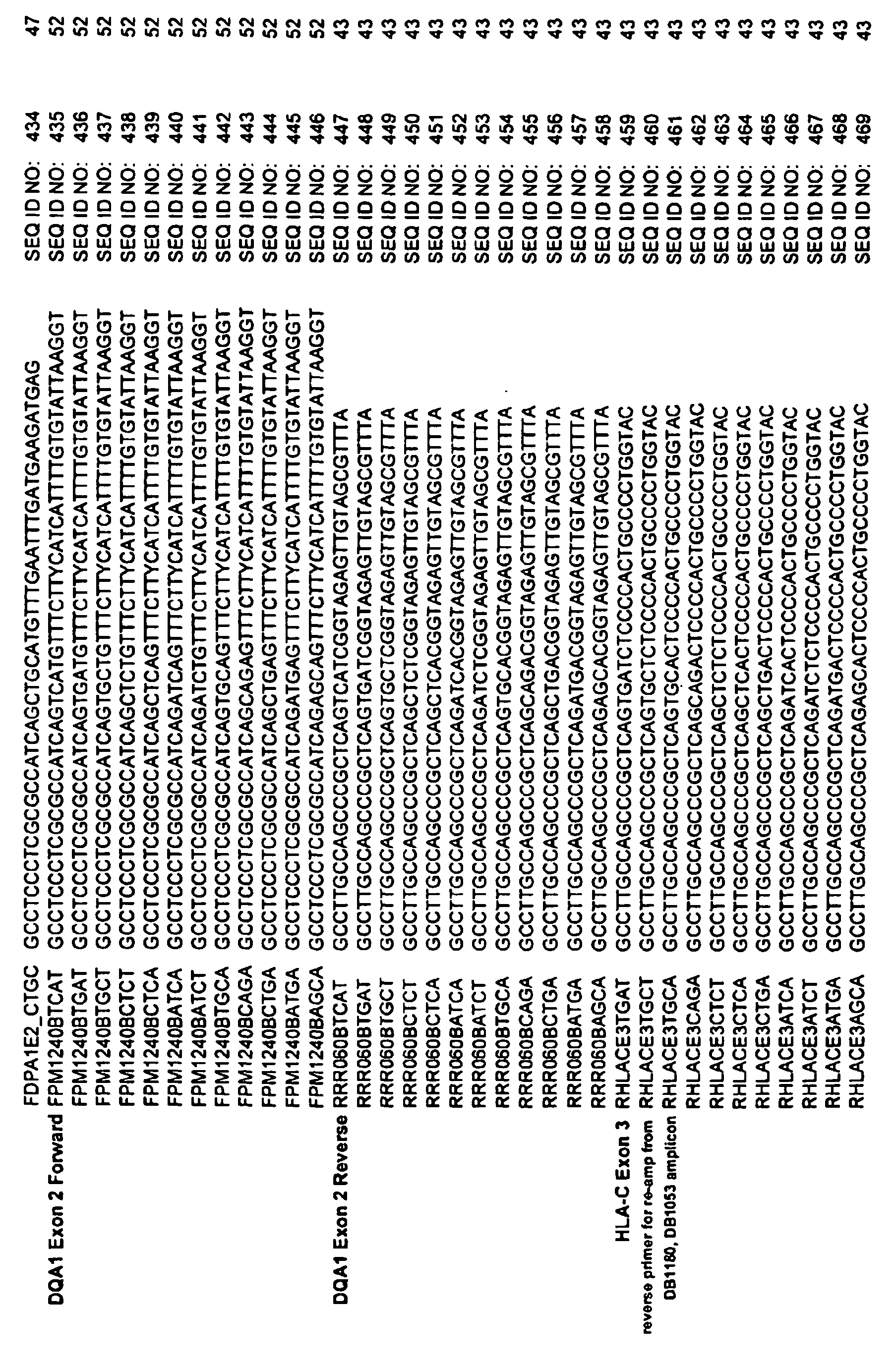

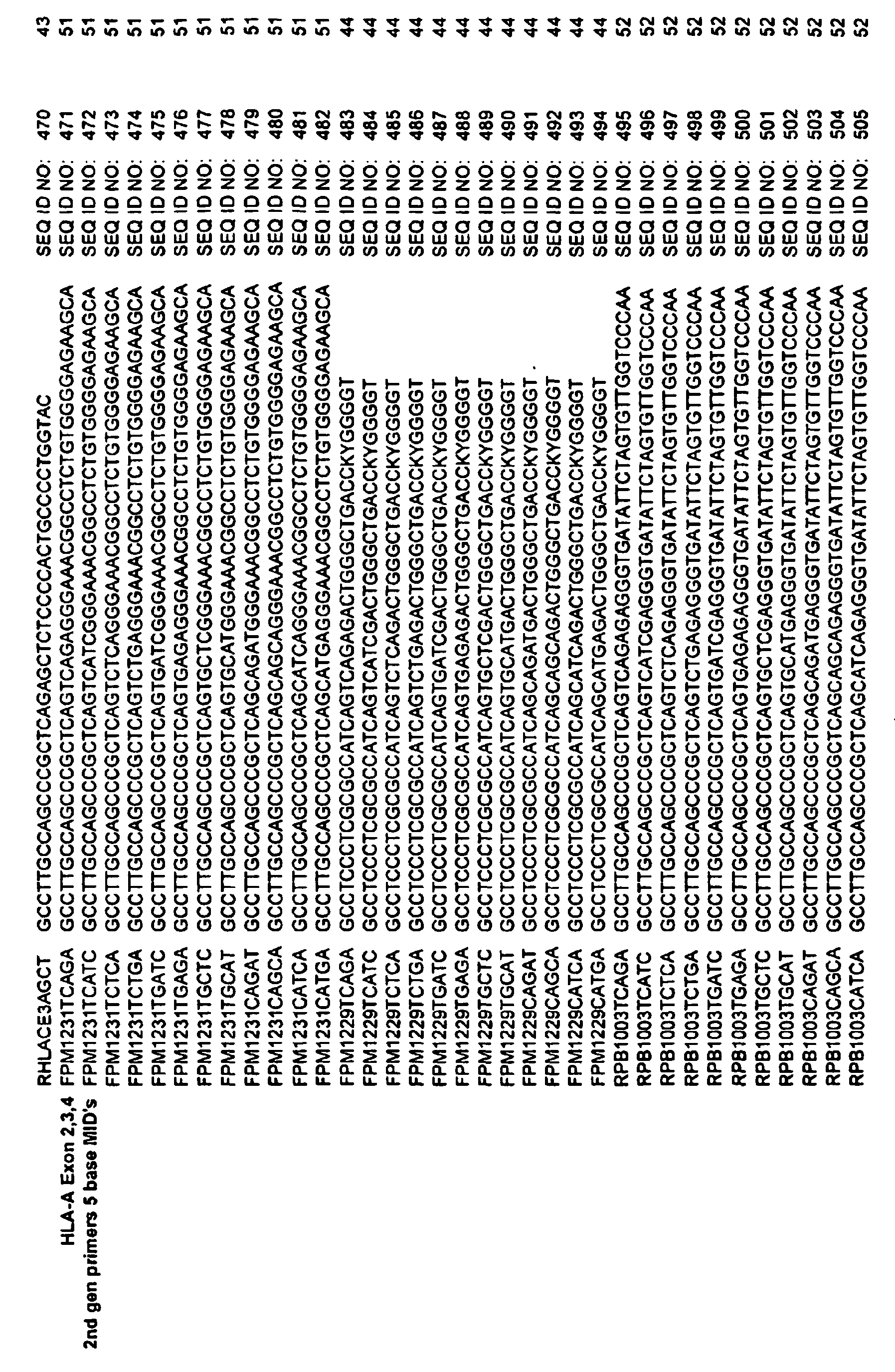

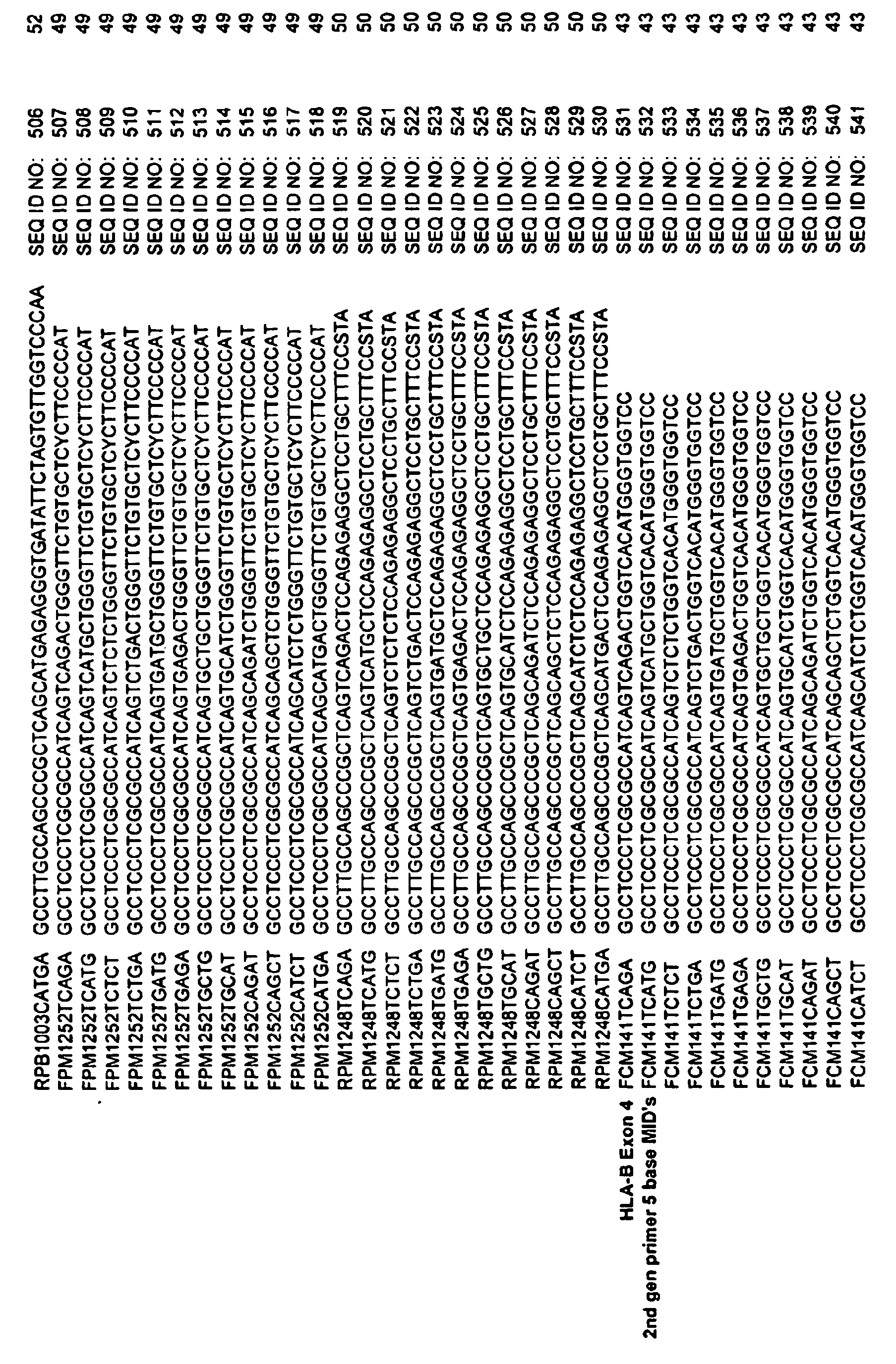

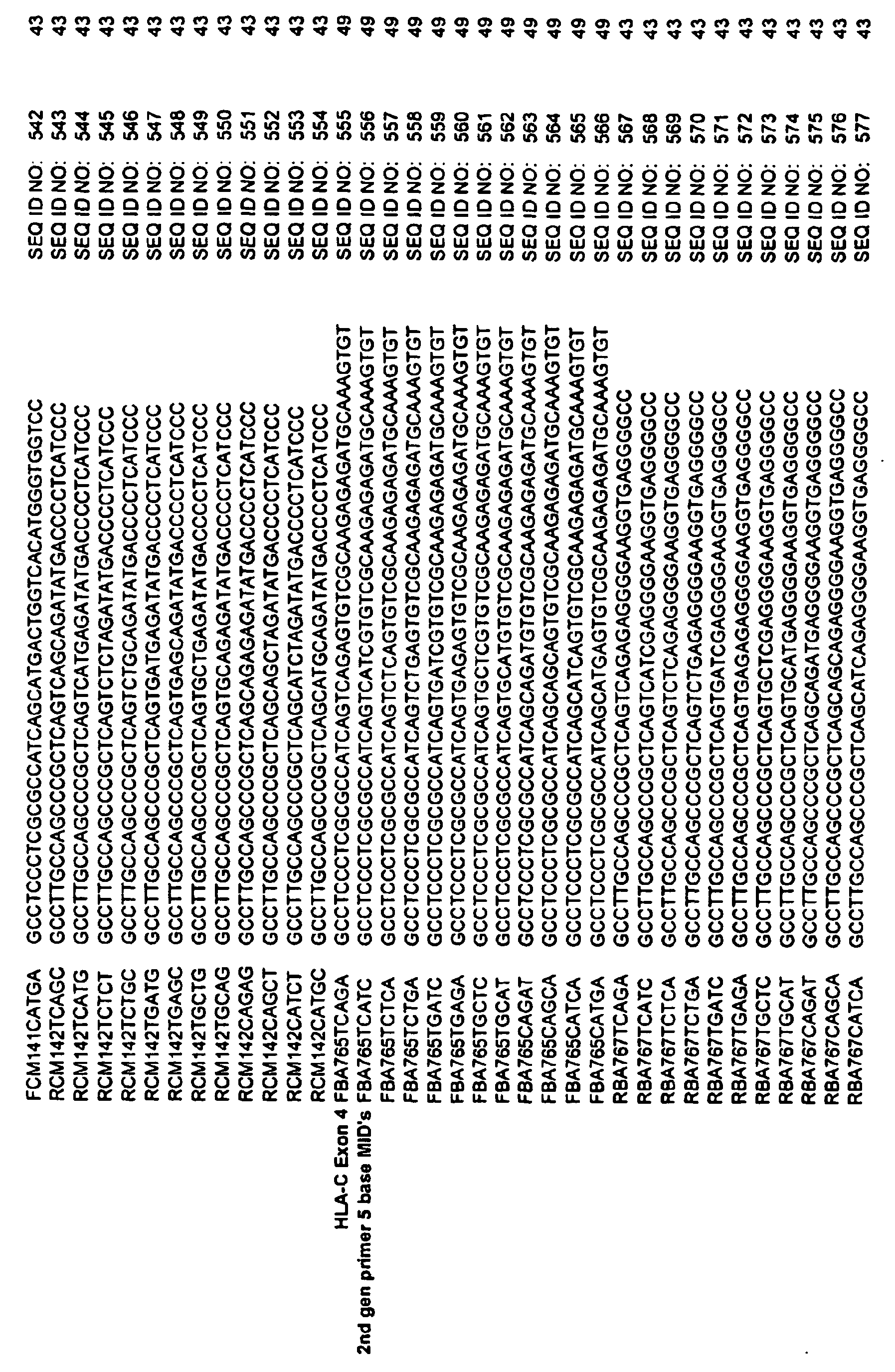

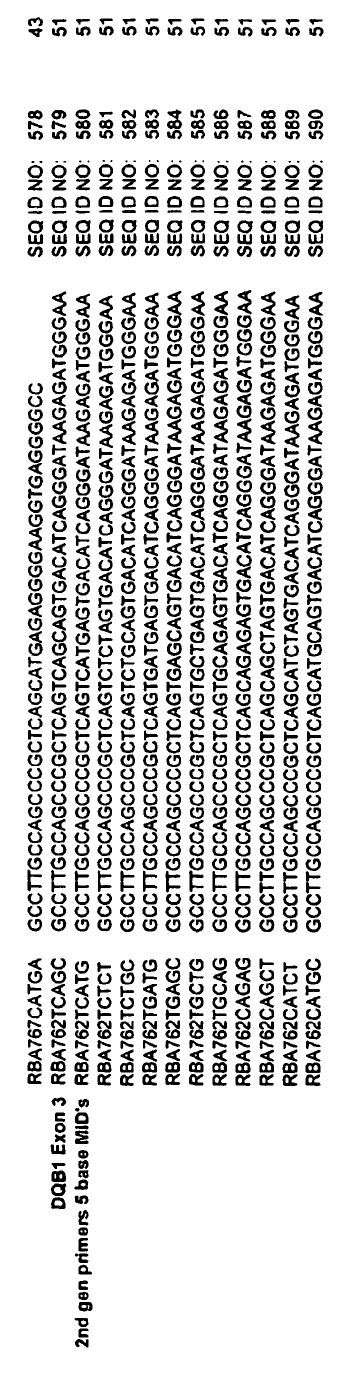

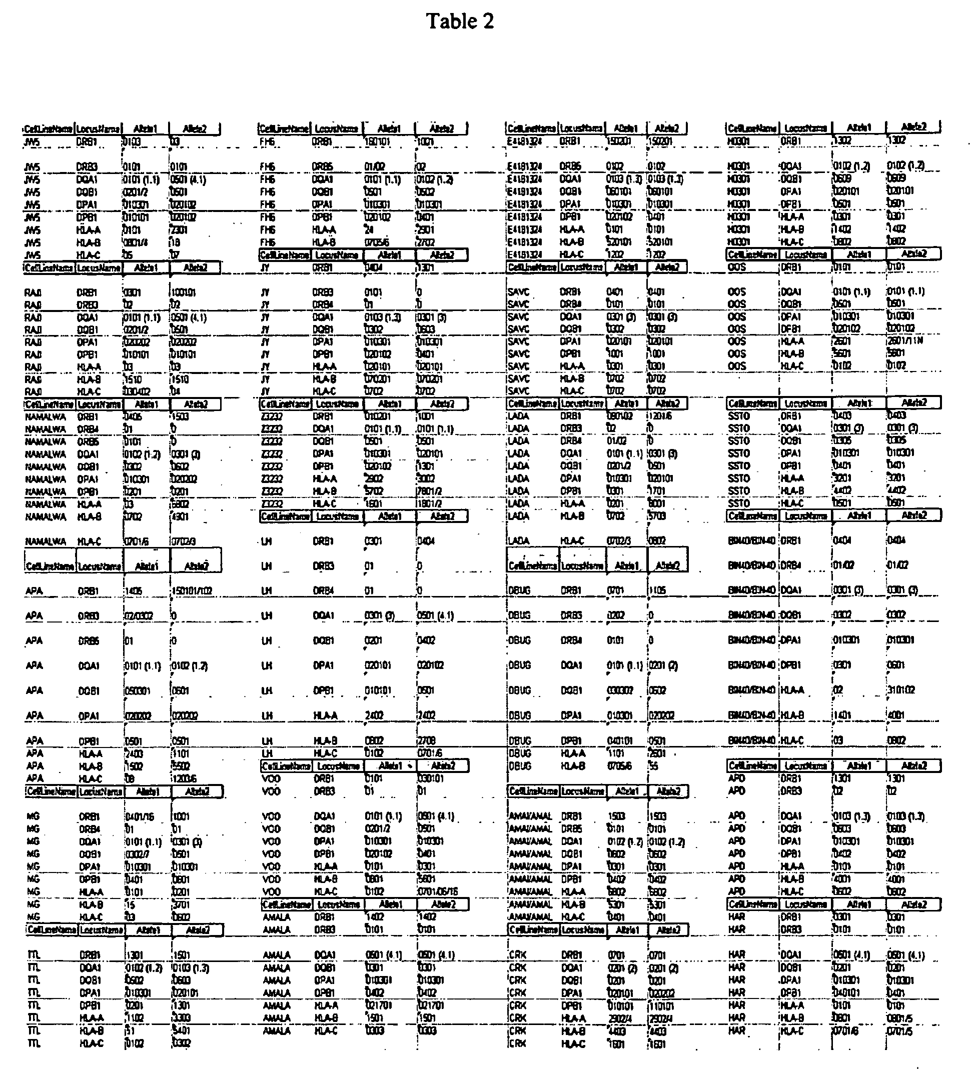

## Claims

1. A method of determining the HLA genotypes for the HLA genes HLA-A, HLA-B, HLA-C, DRB1, DQA1, DQB1, DPA1, and DPB1 for more than one individuals in parallel, the method comprising:
(a) for each individual, amplifying the exons of the HLA-A, HLA-B, HLA-C, DRB1, DQA1, DQB1, DPA1, and DPB1 genes that comprises polymorphic sites to obtain HLA-A, HLA-B, HLA-C, DRB1, DQA1, DQB1, DPA1, and DPB1 amplicons for each individual, wherein each amplification reaction is performed with a forward primer and a reverse primer to amplify an HLA gene exon, where:
(i) the forward primer comprises the following sequences, from 5' to 3': an adapter sequence, a molecular identification sequence, and an HLA-hybridizing sequence, said HLA-hybridizing sequence having 15 to 40 nucleotides in length and being exactly or substantially complementary to the sequence of the HLA gene exon of interest; and
(ii) the reverse primer comprises the following sequences, from 5' to 3':
an adapter sequence, a molecular identification sequence, and an HLA-hybridizing sequence,
said HLA-hybridizing sequence having 15 to 40 nucleotides in length and being exactly or
substantially complementary to the sequence of the HLA gene exon of interest;
(b) pooling HLA amplicons from more than one individual and performing emulsion PCR;
(c) determining the sequence of the HLA-A, HLA-B, HLA-C, DRB1, DQA1, DQB1, DPA1, and DPB 1 amplicon for each individual using pyrosequencing in parallel; and
(d) assigning the HLA alleles to each individual by comparing the sequence of the HLA amplicons to known HLA sequences to determine which HLA alleles are present in the individual.

2. The method of claim 1, wherein the forward primer for obtaining an HLA amplicon has the sequence of an HLA-binding region of a primer set forth in Table 1.

3. The method of claim 2, wherein the forward primer has a sequence of a primer set forth in Table 1.

4. The method of claim 1, wherein the reverse primer for obtaining an HLA amplicon has the sequence of an HLA-binding region of a primer set forth in Table 1.

5. The method of claim 4, wherein the reverse primer has a sequence of a primer set forth in Table 1.

6. The method of claim 1, wherein the forward primer for obtaining an HLA amplicon has the sequence of an HLA-hybridizing region of a primer set forth in Table 1; and the reverse primer for obtaining the HLA amplicon has the sequence of an HLA-hybridizing region of a primer set forth in Table 1.

7. The method of claim 6, wherein the forward primer has an adapter region of a primer set forth in Table 1; and the reverse primer has an adapter region of a primer set forth in Table 1.

8. The method of claim 1, wherein the forward primer has an individual identification tag of a primer set forth in Table 1 and the reverse primer has an individual identification tag of a primer set forth in Table 1.

9. The method of claim 1, wherein the emulsion PCR is performed asymmetrically.

10. A kit comprising primer pairs for obtaining HLA amplicons to determine the HLA genotypes for the HLA genes HLA-A, HLA-B, HLA-C, DRB1, DQA1, DQB1, DPA1, and DPB1 for more than one individuals in parallel, wherein the primer pairs comprise a forward primer and a reverse primer to amplify an HLA gene exon, where: (i) the forward primer comprises the following sequences, from 5' to 3': an adapter sequence, a molecular identification sequence, and an HLA-hybridizing sequence, said HLA-hybridizing sequence having 15 to 40 nucleotides in length and being exactly or substantially complementary to the sequence of the HLA gene exon of interest; and (ii) the reverse primer comprises the following sequences, from 5' to 3': an adapter sequence, a molecular identification sequence, and an HLA-hybridizing sequence, said HLA-hybridizing sequence having 15 to 40 nucleotides in length and being exactly or substantially complementary to the sequence of the HLA gene exon of interest.

11. The kit of claim 10, wherein the primer pairs comprise forward and reverse primers set forth in Table 1.

12. A kit comprising one or more primer pairs, wherein each primer pair comprises a forward primer for obtaining an HLA amplicon that has the sequence of an HLA-binding region of a primer set forth in Table 1; and a reverse primer for obtaining the HLA amplicon that has the sequence of an HLA-binding region of a primer set forth in Table 1.

13. The kit of claim 12, wherein the forward primer has a sequence of a primer set forth in Table 1 and the reverse primer has a sequence of a primer set forth in Table 1.

14. The kit of claim 12, wherein the kit comprises fifteen HLA primer pairs, where the primer pairs amplify exon 2, exon 3, and exon 4 of HLA-A; exon 2, exon 3, and exon 4 of HLA-B; exon 2, exon 3, and exon 4 of HLA-C; exon 2 of DRB1, exon 2 of DPB1, exon 2 of DPA1, exon 2 of DQA1; and exon 2 and exon 3 of DQB1.

## Patentansprüche

1. Verfahren zum Bestimmen der HLA-Genotypen für die HLA-Gene HLA-A, HLA-B, HLA-C, DRB1, DQA1, DQB1, DPA1 und DPB1 parallel für mehr als ein Individuum, wobei das Verfahren Folgendes umfasst:
(a) Amplifizieren der Exons der HLA-A-, HLA-B-, HLA-C-, DRB1-, DQA1-, DQB1-, DPA1- und DPB1-Gene, die polymorphe Stellen umfassen, für jedes Individuum, wodurch man HLA-A-, HLA-B-, HLA-C-, DRB1-, DQA1-, DQB1-, DPA1- und DPB1-Amplicons für jedes Individuum erhält, wobei jede Amplifikation mit einem forward-Primer und einem reverse-Primer zum Amplifizieren eines HLA-Gen-Exons durchgeführt wird, wobei:
(i) der forward-Primer in 5'- bis 3'-Richtung die folgenden Sequenzen umfasst: eine Adapter-Sequenz, eine Sequenz für die molekulare Identifizierung und eine mit HLA hybridisierende Sequenz, wobei die mit HLA hybridisierende Sequenz 15 bis 40 Nukleotide lang ist und vollständig oder im Wesentlichen zu der Sequenz des interessierenden HLA-Gen-Exons komplementär ist; und
(ii) der reverse-Primer in 5'- bis 3'-Richtung die folgenden Sequenzen umfasst: eine Adapter-Sequenz, eine Sequenz für die molekulare Identifizierung und eine mit HLA hybridisierende Sequenz, wobei die mit HLA hybridisierende Sequenz 15 bis 40 Nukleotide lang ist und vollständig oder im Wesentlichen zu der Sequenz des interessierenden HLA-Gen-Exons komplementär ist;
(b) Poolen der HLA-Amplicons von mehr als einem Individuum und Durchführen einer Emulsions-PCR;
(c) Bestimmen der Sequenz des HLA-A-, HLA-B-, HLA-C-, DRB1-, DQA1-, DQB1-, DPA1- und DPB1-Amplicons für jedes Individuum parallel unter Verwendung der Pyrosequenzierung; und
(d) Zuordnen der HLA-Allele zu jedem Individuum durch Vergleich der Sequenz der HLA-Amplicons mit bekannten HLA-Sequenzen, um zu bestimmen, welche HLA-Allele in dem Individuum vorliegen.

2. Verfahren nach Anspruch 1, wobei der forward-Primer für die Gewinnung eines HLA-Amplicons die Sequenz einer HLA-Bindungsregion eines Primers gemäß Tabelle 1 aufweist.

3. Verfahren nach Anspruch 2, wobei der forward-Primer eine Sequenz eines Primers gemäß Tabelle 1 aufweist.

4. Verfahren nach Anspruch 1, wobei der reverse-Primer für die Gewinnung eines HLA-Amplicons die Sequenz einer HLA-Bindungsregion eines Primers gemäß Tabelle 1 aufweist.

5. Verfahren nach Anspruch 4, wobei der reverse-Primer eine Sequenz eines Primers gemäß Tabelle 1 aufweist.

6. Verfahren nach Anspruch 1, wobei der forward-Primer für die Gewinnung eines HLA-Amplicons die Sequenz einer HLA-Hybridisierungsregion eines Primers gemäß Tabelle 1 aufweist; und der reverse-Primer für die Gewinnung des HLA-Amplicons die Sequenz einer HLA-Hybridisierungsregion eines Primers gemäß Tabelle 1 aufweist.

7. Verfahren nach Anspruch 6, wobei der forward-Primer eine Adapterregion eines Primers gemäß Tabelle 1 aufweist und der reverse-Primer eine Adapterregion eines Primers gemäß Tabelle 1 aufweist.

8. Verfahren nach Anspruch 1, wobei der forward-Primer einen individuellen Identifikations-Tag eines Primers gemäß Tabelle 1 aufweist und der reverse-Primer einen individuellen Identifikations-Tag eines Primers gemäß Tabelle 1 aufweist.

9. Verfahren nach Anspruch 1, wobei die Emulsions-PCR asymmetrisch durchgeführt wird.

10. Kit, umfassend Primer-Paare zur Gewinnung von HLA-Amplicons zur Bestimmung der HLA-Genotypen für die HLA-Gene HLA-A, HLA-B, HLA-C, DRB1, DQA1, DQB1, DPA1 und DPB1 parallel für mehr als ein Individuum, wobei die Primer-Paare einen forward-Primer und einen reverse-Primer zum Amplifizieren eines HLA-Gen-Exons umfassen, wobei: (i) der forward-Primer in 5'- bis 3'-Richtung die folgenden Sequenzen umfasst: eine Adapter-Sequenz, eine Sequenz für die molekulare Identifizierung und eine mit HLA hybridisierende Sequenz, wobei die mit HLA hybridisierende Sequenz 15 bis 40 Nukleotide lang ist und vollständig oder im Wesentlichen zu der Sequenz des interessierenden HLA-Gen-Exons komplementär ist und (ii) der reverse-Primer in 5'- bis 3'-Richtung die folgenden Sequenzen umfasst: eine Adapter-Sequenz, eine Sequenz für die molekulare Identifizierung und eine mit HLA hybridisierende Sequenz, wobei die mit HLA hybridisierende Sequenz 15 bis 40 Nukleotide lang ist und vollständig oder im Wesentlichen zu der Sequenz des interessierenden HLA-Gen-Exons komplementär ist.

11. Kit nach Anspruch 10, wobei die Primer-Paare die forward- und reverse-Primer gemäß Tabelle 1 umfassen.

12. Kit, umfassend ein oder mehr Primer-Paare, wobei jedes Primer-Paar einen forward-Primer zur Gewinnung eines HLA-Amplicons mit der Sequenz einer HLA-Bindungsregion eines Primers gemäß Tabelle 1 und einen reverse-Primer zur Gewinnung des HLA-Amplicons mit der Sequenz einer HLA-Bindungsregion eines Primers gemäß Tabelle 1 umfasst.

13. Kit nach Anspruch 12, wobei der forward-Primer eine Sequenz eines Primers gemäß Tabelle 1 aufweist und der reverse-Primer eine Sequenz eines Primers gemäß Tabelle 1 aufweist.

14. Kit nach Anspruch 12, wobei das Kit fünfzehn HLA-Primer-Paare umfasst, wobei die Primer-Paare Exon 2, Exon 3 und Exon 4 von HLA-A; Exon 2, Exon 3 und Exon 4 von HLA-B; Exon 2, Exon 3 und Exon 4 von HLA-C; Exon 2 von DRB1, Exon 2 von DPB1, Exon 2 von DPA1, Exon 2 von DQA1; und Exon 2 und Exon 3 von DQB1 amplifizieren.

## Revendications

1. Procédé de détermination des génotypes HLA pour les gènes HLA HLA-A, HLA-B, HLA-C, DRB1, DQA1, DQB1, DPA1 et DPB1 pour plus d'un individu en parallèle, le procédé comprenant :
(a) pour chaque individu, l'amplification des exons des gènes HLA-A, HLA-B, HLA-C, DRB1, DQA1, DQB1, DPA1 et DPB1 qui comprennent des sites polymorphes pour obtenir des amplicons HLA-A, HLA-B, HLA-C, DRB1, DQA1, DQB1, DPA1 et DPB1 pour chaque individu, chaque réaction d'amplification étant mise en oeuvre avec une amorce sens et avec une amorce antisens pour amplifier un exon de gène HLA, procédé dans lequel :
(i) l'amorce sens comprend les séquences suivantes, de 5' à 3' : une séquence adaptateur, une séquence d'identification moléculaire et une séquence d'hybridation au HLA, ladite séquence d'hybridation au HLA possédant une longueur de 15 à 40 nucléotides et étant exactement ou essentiellement complémentaire à la séquence de l'exon d'intérêt du gène HLA ; et
(ii) l'amorce antisens comprend les séquences suivantes, de 5' à 3' : une séquence adaptateur, une séquence d'identification moléculaire et une séquence d'hybridation au HLA, ladite séquence d'hybridation au HLA possédant une longueur de 15 à 40 nucléotides et étant exactement ou essentiellement complémentaire à la séquence de l'exon d'intérêt du gène HLA ;
(b) rassembler des amplicons HLA provenant de plus d'un individu et procéder à une PCR en émulsion ;
(c) déterminer la séquence de l'amplicon HLA-A, HLA-B, HLA-C, DRB1, DQA1, DQB1, DPA1 et DPB1 pour chaque individu en utilisant un pyroséquençage en parallèle ; et
(d) attribuer les allèles HLA à chaque individu en comparant la séquence des amplicons HLA à des séquences HLA connues afin de déterminer les allèles HLA qui sont présents dans l'individu.

2. Procédé selon la revendication 1, dans lequel l'amorce sens pour l'obtention d'un amplicon HLA possède la séquence d'une région de liaison au HLA d'une amorce reprise dans le tableau 1.

3. Procédé selon la revendication 2, dans lequel l'amorce sens possède une séquence d'une amorce reprise dans le tableau 1.

4. Procédé selon la revendication 1, dans lequel l'amorce antisens pour l'obtention d'un amplicon HLA possède la séquence d'une région de liaison au HLA d'une amorce reprise dans le tableau 1.

5. Procédé selon la revendication 4, dans lequel l'amorce antisens possède une séquence d'une amorce reprise dans le tableau 1.

6. Procédé selon la revendication 1, dans lequel l'amorce sens pour l'obtention d'un amplicon HLA possède la séquence d'une région d'hybridation au HLA d'une amorce reprise dans le tableau 1 et l'amorce antisens pour l'obtention d'un amplicon HLA possède la séquence d'une région d'hybridation au HLA d'une amorce reprise dans le tableau 1.

7. Procédé selon la revendication 6, dans lequel l'amorce sens possède une région adaptateur d'une amorce reprise dans le tableau 1 et l'amorce antisens possède une région adaptateur d'une amorce reprise dans le tableau 1.

8. Procédé selon la revendication 1, dans lequel l'amorce sens possède une étiquette d'identification individuelle d'une amorce reprise dans le tableau 1 et l'amorce antisens possède une étiquette d'identification individuelle d'une amorce reprise dans le tableau 1.

9. Procédé selon la revendication 1, dans lequel la PCR en émulsion est mise en oeuvre de manière asymétrique.

10. Nécessaire comprenant des paires d'amorces pour l'obtention d'amplicons HLA afin de déterminer des génotypes HLA pour les gènes HLA HLA-A, HLA-B, HLA-C, DRB1, DQA1, DQB1, DPA1 et DPB1 pour plus d'un individu en parallèle, les paires d'amorces comprenant une amorce sens et une amorce antisens pour l'amplification d'un exon du gène HLA, dans lequel : (i) l'amorce sens comprend les séquences suivantes, de 5' à 3' : une séquence adaptateur, une séquence d'identification moléculaire et une séquence d'hybridation au HLA, ladite séquence d'hybridation au HLA possédant une longueur de 15 à 40 nucléotides et étant exactement ou essentiellement complémentaire à la séquence de l'exon d'intérêt du gène HLA ; et (ii) l'amorce antisens comprend les séquences suivantes, de 5' à 3' : une séquence adaptateur, une séquence d'identification moléculaire et une séquence d'hybridation au HLA, ladite séquence d'hybridation au HLA possédant une longueur de 15 à 40 nucléotides et étant exactement ou essentiellement complémentaire à la séquence de l'exon d'intérêt du gène HLA.

11. Nécessaire selon la revendication 10, dans lequel les paires d'amorces comprennent des amorces sens et des amorces antisens reprises dans le tableau 1.

12. Nécessaire comprenant une ou plusieurs paires d'amorces, chaque paire d'amorces comprenant une amorce sens pour l'obtention d'un amplicon HLA qui possède la séquence d'une région de liaison au HLA d'une amorce reprise dans le tableau 1 et une amorce antisens pour l'obtention de l'amplicon HLA qui possède la séquence d'une région de liaison au HLA d'une amorce reprise dans le tableau 1.

13. Nécessaire selon la revendication 12, dans lequel l'amorce sens possède une séquence d'une amorce reprise dans le tableau 1 et l'amorce antisens possède une séquence d'une amorce reprise dans le tableau 1.

14. Nécessaire selon la revendication 12, dans lequel le nécessaire comprend 15 paires d'amorces HLA, les paires d'amorces amplifiant l'exon 2, l'exon 3 et l'exon 4 de HLA-A, l'exon 2, l'exon 3 et l'exon 4 de HLA-B, l'exon 2, l'exon 3 et l'exon 4 de HLA-C, l'exon 2 de DRB1, l'exon 2 de DPB1, l'exon 2 de DPA1, l'exon 2 de DQA1 et l'exon 2 et l'exon 3 de DQB1.
